# EUROPEAN PATENT APPLICATION

(11) **EP 4 442 276 A1**
(43) Date of publication of application: **09.10.2024**
(21) Application number: 23305528.4
(22) Date of filing: 07.04.2023
(51) Int. Cl.: A61K 39/395, A61P 31/14, C07K 16/10

(54) **COMBINED ANTIBODIES AGAINST SARBECOVIRUSES AND USES THEREOF**

(71) Applicant: INSTITUT PASTEUR, 75724 Paris Cedex 15 (FR)
(72) Inventor: Mouquet, Hugo, 75724 Paris Cedex 15 (FR); Planchais, Cyril, 75724 Paris Cedex 15 (FR); Schwartz, Olivier, 75724 Paris Cedex 15 (FR); Bruel, Timothée, 75724 Paris Cedex 15 (FR)
(74) Representative: Cabinet Nony

(57) **Abstract**

The invention relates to combined antibodies against Sarbecoviruses, in particular Severe Acute Respiratory Syndrome-related Coronavirus 2 (SARS-CoV-2), in particular human neutralizing monoclonal antibodies against Severe Acute Respiratory Syndrome-related Coronavirus 2 (SARS-CoV-2).

## Description

### FIELD OF THE INVENTION

The invention relates to antibodies, and antigen-binding fragments thereof, against a Sarbecovirus, preferably Severe Acute Respiratory Syndrome-related Coronavirus 2 (SARS-CoV-2).

In particular, the invention relates to human neutralizing monoclonal antibodies against a Sarbecovirus, preferably Severe Acute Respiratory Syndrome-related Coronavirus 2 (SARS-CoV-2) and their use for the diagnosis, monitoring, prevention, and treatment of a Sarbecovirus infection and related disease, in particular SARS-CoV-2 infection and associated disease (COVID-19).

### BACKGROUND OF THE INVENTION

The pandemic caused by emerging Severe Acute Respiratory Syndrome Coronavirus 2 (SARS-CoV-2), which causes Coronavirus Disease-2019 (COVID-19) and accounts to date for nearly 762 million infection cases and 6.9 million deaths worldwide (https://www.who.int/), presents a serious global public health emergency in urgent need for prophylactic and therapeutic interventions.

Coronaviruses are enveloped, positive-sense, single-stranded RNA viruses that infect humans and mammals. Coronaviruses genomes encode non-structural polyprotein and structural proteins, including the homotrimeric spike (S) glycoprotein, envelope (E), membrane (M) and nucleocapsid (N) proteins. Several coronaviruses are pathogenic to human, leading to varying degrees of symptoms severity (Cui et al., Nat Rev Microbiol. 2019 Mar; 17(3):181-92). The betacoronavirus genus (Beta-CoV or β-CoV) which is divided in 4 lineages or groups (A, B, C, D) comprises the highly human-pathogenic coronaviruses in group B/C. Beta-CoV group B/C includes the severe acute respiratory syndrome coronavirus (SARS-CoV or SARS-CoV-1) that emerged in China in 2002, the Middle East respiratory syndrome coronavirus (MERS-CoV), first detected in Saudi Arabia in 2012, and the new coronavirus named SARS-CoV-2 that causes COVID-19, isolated in China in 2019 (SARS-CoV-2 isolate Wuhan-Hu-1), in association with cases of severe acute respiratory syndrome (Peiris et al., Nat Med., 2004 Dec;10(12 Suppl):S88-97; Zaki et al., N Engl J Med., 2012 Nov 8;367(19):1814-20 ; Lee et al., BMC Infect Dis. 2017 Jul 14;17(1):498; Zhu N et al., N Engl J Med., 2020 Jan 24). The β-CoV sub-genus Sarbecovirus includes human-pathogenic SARS-CoV-1 and SARS-CoV-2. In contrast, Beta-CoV group A includes HCoV-OC43 and HCoV-HKU1 which can cause the common cold.

Antibodies developing in response to SARS-CoV-2 infection and vaccination are essential for long-term protection against COVID-19. Human neutralizing SARS-CoV-2 antibodies appear to play a key role in the control of COVID-19 infection and represent promising immunotherapeutic tools for treating SARS-CoV-2 infected humans with mild-to-moderate disease. Decoding antibody responses in COVID-19 is fundamental in understanding the basic mechanisms of humoral immunity to the SARS-CoV-2 Spike protein (SARS-CoV-2-S), the target of neutralizing antibodies, but also to develop effective vaccine and monoclonal antibody-based immunotherapy strategies.

The Spike glycoprotein has key roles in the viral cycle, as it is involved in receptor recognition, virus attachment and entry, and is thus a crucial determinant of host tropism and transmission capacity. SARS-CoV-2 cellular entry depends on binding between the viral Spike protein receptor-binding domain (RBD) and the angiotensin converting enzyme 2 (ACE2) target receptor. Binding with ACE2 triggers a cascade of cell membrane fusion events for viral entry. Each S protomer consists of two subunits that are cleaved by proteases: a globular S1 domain and the N-terminal region, and the membrane-proximal S2 and transmembrane domains. Determinants of host range and cellular tropisms are found in the RBD within the S1 domain, while mediators of membrane fusion have been identified within the S2 domain. Anti-SARS-CoV-2 antibody neutralizing potency is determined by competition with ACE2 receptor for RBD binding.

Antibodies rapidly develop in response to SARS-CoV-2 infection, including neutralizing antibodies recognizing distinct S protein regions. The RBD is the primary target of neutralizing antibodies including potent neutralizers, but the NTD and S2 fusion peptide and stem regions also contain neutralizing epitopes. SARS-CoV-2 neutralizing IgA antibodies are detected as early as a week after onset of symptoms, contribute to seroneutralization and can be as potent as IgGs. Neutralizing antibodies are the main correlate of protection for COVID-19 vaccines. Still, SARS-CoV-2 spike-specific antibodies, including non-neutralizers, can exert antiviral Fc-dependent effector functions important for *in vivo* protection i.e., antibody-dependent cellular cytotoxicity (ADCC), and phagocytosis (ADCP).

Since mid-2020, emerging variants of SARS-CoV-2 with increased transmissibility and/or reduced sensitivity to neutralizing antibodies due to predicted mutations in the Spike protein, in particular its receptor-binding region (RBD), were reported in several countries and are currently spreading worldwide. The first variant reported was in UK (lineage B.1.1.7; notable mutations N501Y, 69-70del, P681H); then in South Africa (SA) (lineage B.1.351; notable mutations N501Y, E484K, K417N) and Brazil (BR) (lineage P.1; notable mutations N501Y, E484K, K417T). Some monoclonal and serum-derived antibodies are reported to be from 10 to 60 time less effective in neutralizing virus bearing the E484K mutation (SARS-CoV-2 variants SA and BR). Some vaccines might see their efficacy reduced against these variants.

Since then, new emerging variants of SARS-CoV-2 are spreading worldwide, including lineages of Variants of Concerns (VOCs) such as the ones cited below, or VOCs comprising the same mutations in the spike proteins responsible for increased affinity to hACE2 and potential immune escape:
Alpha (B.1.1.7) N501Y; A570D; P681H; T716I; S982A; D1118H
Beta (B.1.351) D80A; D215G; K417N; E484K; N501Y; A701V
Gamma (P.1) L18F; T20 N; P26S; D138Y; R190S; K417T; E484K; N501Y; H655Y; T1027I
Delta (B.1.617.2) T19R; L452R; T478K; P681R; D950N
Omicron (BA.1 sublineage) (B. 1.1.529) A67V, H69-, V70-, T95I, G142-, V143-, Y144-, Y145D, N211-, L212I, G339D, S371L, S373P, S375F, K417N, N440K, G446S, S477N, T478K, E484A,
Q493R, G496S, Q498R, N501Y, T547K, D614G, H655Y, N679K, P681H, N764K, D796Y, N856K, Q954H, N969K, L981F
Omicron (BA.2 sublineage) G339D; F367V; S371F; S373P; S375F; T376A; D405N; R408S; K417N; N440K; S477N; T478K; E484A; Q493R; Q498R; N501Y; Y505H
Omicron (BA.2.75 sublineage) G339H; F367V; S371F; S373P; S375F; T376A; D405N; R408S; K417N; N440K; G446S, N460K, S477N; T478K; E484A; Q493R; Q498R; N501Y; Y505H
Omicron (BA.2.75.2 sublineage) G339H; R346T; F367V; S371F; S373P; S375F; T376A; D405N; R408S; K417N; N440K; G446S, N460K, S477N; T478K; E484A; F486S; R493Q; Q498R; N501Y; Y505H
Omicron (BA.4 and BA.5 sublineages) G339D; S371F; S373P; S375F; T376A; D405N; K417N; N440K; L452R; S477N; T478K; E484A; F486V; Q498R; N501Y; Y505HOmicron (BA.4.6 sublineage) G339D; R346T; S371F; S373P; S375F; T376A; D405N; K417N; N440K; L452R; S477N; T478K; E484A; F486V; Q498R; N501Y; Y505H
Omicron (BF.7 sublineage) G339D; R346T; S371F; S373P; S375F; T376A; D405N; R408S; K417N; N440K; L452R; S477N; T478K; E484A; F486V; Q498R; N501Y; Y505H
Omicron (BO.1 sublineage) G339D; S371F; S373P; S375F; T376A; D405N; R408S; K417N; N440K; K444T; L452R; N460K; S477N; T478K; E484A; F486V; Q498R; N501Y; Y505H
Omicron (BQ.1.1 sublineage) G339D; R346T; S371F; S373P; S375F; T376A; D405N; R408S; K417N; N440K; K444T; L452R; N460K; S477N; T478K; E484A; F486V; Q498R; N501Y; Y505H
Omicron (XBB.1 sublineage) G339D; R346T; F367V; S371F; S373P; S375F; T376A; D405N; R408S; K417N; N440K; V445P; G446S; N460K; S477N; T478K; E484A; F486S; F490S; R493Q; Q498R; N501Y; Y505H
Omicron (XBB.1.5 sublineage) G339H; R346T; F367V; S371F; S373P; S375F; T376A; D405N; R408S; K417N; N440K; V445P; G446S; N460K; S477N; T478K; E484A; F486P; F490S; R493Q; Q498R; N501Y; Y505H.

To develop prophylactic and therapeutic approaches specific to a Sarbecovirus infection, in particular SARS-CoV-2, there is a need for neutralizing antibodies against Sarbecoviruses (e.g. SARS-CoV-2), in particular human neutralizing antibodies against Sarbecoviruses (e.g. SARS-CoV-2) including human antibodies capable of neutralizing Sarbecovirus (e.g.SARS-CoV-2) variants, especially the above VOCs and variants of such VOCs and VOCs having a combination of the above mutations.

The challenge is to provide monoclonal antibodies, alone or in combination, which retain efficient neutralizing properties to confer protection to individuals at risk of developing a Sarbecovirus-related disease, especially SARS, from present and future VOCs. While it cannot be expected that a universal Sarbecovirus (e.g. SARS-CoV2) anti-Spike monoclonal antibody (mAb) would be such that it would keep sufficient efficacy against most VOCs and future VOCs, it is a goal to provide antibodies and antigen-binding fragments thereof, in particular mAbs, that are consistent in neutralizing the majority of these VOCs and VOCs comprising different combinations of these mutations; so that it remains useful overtime to prevent or treat SARS.

Immunotherapies based on Sarbecovirus neutralizing antibodies, especially SARS-CoV-2 neutralizing antibodies, have been rapidly explored, and this led to the clinical use of several mAbs alone or in bi-therapies.

Highly potent human SARS-CoV-2 neutralizing antibodies isolated so far, including those tested or used in clinics, mainly target the RBD and can prevent or protect animals from infection in preclinical models. However, viral variants with spike mutations conferring resistance to antibody neutralization emerged during the pandemics and annihilated some of these therapies. The search for broadly neutralizing monoclonal antibodies (mAbs) is pursued. Novel antibodies active against all VOCs, have been described.

In particular, WO2022228827 A1 and Planchais et al. ("Potent human broadly SARS-CoV-2-neutralizing IgA and IgG antibodies effective against Omicron BA.1 and BA.2"; J. Exp. Med. 2022; 219(7):e20220638) describe a selection human monoclonal antibodies directed against the receptor-binding domain (RBD) of the Spike protein.

This further includes human antibodies directed against the RBD such as SA55, as reported in Cao et al. ("Rational identification of potent and broad sarbecovirus-neutralizing antibody cocktails from SARS convalescents"; Cell Reports 41, 2022).

This also includes novel, non-RBD binding, antibody molecules targeting more conserved epitopes on the S2 subunit, located either on the fusion peptide or the S2 stem helix, as reported in Mast et al. ("Highly synergistic combinations of nanobodies that target SARS-CoV-2 and are resistant to escape"; eLife 2021; 10:e73027) or Pinto et al. ("Broad betacoronavirus neutralization by a stem helix-specific human antibody"; Science 373, 1109-1116, 2021) or Bianchini et al. ("Human neutralizing antibodies to cold linear epitopes and subdomain1 of the SARS-CoV-2 spike glycoprotein"; Sci. Immunol. 8, eade0958, 2023) or Dacon et al. ("Rare, convergent antibodies targeting the stem helix broadly neutralize diverse betacoronaviruses"; Cell Host & Microbe 31, 97-111, 2023) .

Other non-RBD binding antibody molecules targeting novel epitopes were reported, in or around the heptad repeat 2 (HR2) region on the S2 subunit, as shown with single-domain antibodies in De Cae et al. ("Ultrapotent SARS coronavirus-neutralizing single-domain antibodies that bind a conserved membrane proximal epitope of the spike"; Preprint; doi: https://doi.org/10.1101/2023.03.10.531533).

There is thus a need to provide antibody molecules, such as mAbs, that are consistent in neutralizing the majority of these VOCs and VOCs comprising different combinations of these mutations is enormous.

There is also a need for repositioning existing antibody molecules, in particular existing monoclonal antibodies, which have already been developed in clinic, but which may now become less neutralizing toward novel VOCs.

There is also a need for clinical validation of novel antibody combinations, especially in the context of treatment and prophylaxis.

In particular, there is a need for preventing the emergence or re-emergence of Sarbecovirus variants which may become resistant to existing drug, such as monoclonal antibodies.

There is also a need for identifying novel epitopes against emerging VOCs, or alternatively for identifying novel combinations of epitopes.

In particular there is a need for identifying novel epitopes against emerging VOCs, which are non-RBD binding, and/or which may be capable of binding specifically epitopes outside of the RBD region of the Spike protein

The present invention fulfills this need and provides specific antibodies and antigen-binding fragments thereof, in particular human monoclonal antibodies with retained potency across majors VOCs displaying such mutations.

### SUMMARY OF THE INVENTION

According to a **first main embodiment,** the invention relates to a combination of:
(i) An isolated antibody, or antigen-binding fragment thereof, directed against the viral Spike protein heptad repeat 2 (HR2) of a Sarbecovirus, in particular of Severe Acute Respiratory Syndrome-related Coronavirus 2 (SARS-CoV-2); and
(ii) An isolated antibody, or antigen-binding fragment thereof, directed against the viral Spike protein receptor binding-domain (RBD) of a Sarbecovirus, in particular of Severe Acute Respiratory Syndrome-related Coronavirus 2 (SARS-CoV-2);
characterized in that the isolated antibody or antigen-binding fragment thereof directed against the viral Spike protein heptad repeat 2 (HR2) of a Sarbecovirus, binds specifically to a polypeptide comprising **SEQ ID NO: 26** within the polypeptide **SEQ ID NO: 65.**

According to particular embodiments of said main embodiment, the isolated antibody, or antigen-binding fragment thereof, directed against the viral Spike protein heptad repeat 2 (HR2) of a Sarbecovirus, is characterized in that it comprises:
- a heavy chain variable domain comprising all three of: a heavy chain CDR1 of SEQ ID NO: 1, a heavy chain CDR2 of SEQ ID NO: 2 and a heavy chain CDR3 of SEQ ID NO: 3; and a light chain variable domain comprising all three of: a light chain CDR1 of SEQ ID NO: 4, a light chain CDR2 of SEQ ID NO: 5 and a light chain CDR3 of SEQ ID NO: 6.

According to particular embodiments of said main embodiment, the combination is characterized in that the isolated antibody, or antigen-binding fragment thereof, directed against the viral Spike protein heptad repeat 2 (HR2) of a Sarbecovirus, comprises a heavy chain comprising the amino acid sequence of **SEQ ID NO: 15** and a light chain comprising the amino acid sequence of **SEQ ID NO: 16.**

According to particular embodiments of said main embodiment, the combination is characterized in that the antibody, or antigen-binding fragment thereof, directed against the viral Spike protein heptad repeat 2 (HR2) of SARS-Cov-2 binds specifically to a polypeptide comprising **SEQ ID NO: 26** within the polypeptide **SEQ ID NO: 65.**

According to particular embodiments of said main embodiment, the combination is characterized in that the isolated antibody, or antigen-binding fragment thereof, directed against the viral Spike protein receptor binding-domain (RBD) of a Sarbecovirus, comprises:
- a heavy chain variable domain comprising all three of: a heavy chain CDR1 of SEQ ID NO: 29, a heavy chain CDR2 of SEQ ID NO:30 and a heavy chain CDR3 of SEQ ID NO: 31; and a light chain variable domain comprising all three of: a light chain CDR1 of SEQ ID NO: 32, a light chain CDR2 of SEQ ID NO: 33 and a light chain CDR3 of SEQ ID NO: 34; or
- a heavy chain variable domain comprising all three of: a heavy chain CDR1 of SEQ ID NO: 35, a heavy chain CDR2 of SEQ ID NO:36 and a heavy chain CDR3 of SEQ ID NO: 37; and a light chain variable domain comprising all three of: a light chain CDR1 of SEQ ID NO: 38, a light chain CDR2 of SEQ ID NO: 39 and a light chain CDR3 of SEQ ID NO: 40; or
- a heavy chain variable domain comprising all three of: a heavy chain CDR1 of SEQ ID NO: 41, a heavy chain CDR2 of SEQ ID NO: 42 and a heavy chain CDR3 of SEQ ID NO: 43; and a light chain variable domain comprising all three of: a light chain CDR1 of SEQ ID NO: 44, a light chain CDR2 of SEQ ID NO: 45 and a light chain CDR3 of SEQ ID NO: 46; or
- a heavy chain variable domain comprising all three of: a heavy chain CDR1 of SEQ ID NO: 47, a heavy chain CDR2 of SEQ ID NO: 48 and a heavy chain CDR3 of SEQ ID NO: 49; and a light chain variable domain comprising all three of: a light chain CDR1 of SEQ ID NO: 50, a light chain CDR2 of SEQ ID NO: 51 and a light chain CDR3 of SEQ ID NO: 52; or
- a heavy chain variable domain comprising all three of: a heavy chain CDR1 of SEQ ID NO: 53, a heavy chain CDR2 of SEQ ID NO: 54 and a heavy chain CDR3 of SEQ ID NO: 55; and a light chain variable domain comprising all three of: a light chain CDR1 of SEQ ID NO: 56, a light chain CDR2 of SEQ ID NO: 57 and a light chain CDR3 of SEQ ID NO: 58; or
- a heavy chain variable domain comprising all three of: a heavy chain CDR1 of SEQ ID NO: 59, a heavy chain CDR2 of SEQ ID NO:60 and a heavy chain CDR3 of SEQ ID NO: 61; and a light chain variable domain comprising all three of: a light chain CDR1 of SEQ ID NO: 62, a light chain CDR2 of SEQ ID NO: 63 and a light chain CDR3 of SEQ ID NO: 64.

According to particular embodiments of said main embodiment, the combination is characterized in that the (ii) isolated antibody, or antigen-binding fragment thereof, directed against the viral Spike protein receptor binding-domain (RBD) of a Sarbecovirus, comprises:
- heavy chain variable domain comprising all three of: a heavy chain CDR1 of SEQ ID NO: 29, a heavy chain CDR2 of SEQ ID NO:30 and a heavy chain CDR3 of SEQ ID NO: 31; and a light chain variable domain comprising all three of: a light chain CDR1 of SEQ ID NO: 32, a light chain CDR2 of SEQ ID NO: 33 and a light chain CDR3 of SEQ ID NO: 34.

According to particular embodiments of said main embodiment, the combination is characterized in that the isolated antibody, or antigen-binding fragment thereof, directed against the viral Spike protein receptor binding-domain (RBD) of a Sarbecovirus, comprises:
- a heavy chain variable domain comprising all three of: a heavy chain CDR1 of SEQ ID NO: 35, a heavy chain CDR2 of SEQ ID NO:36 and a heavy chain CDR3 of SEQ ID NO: 37; and a light chain variable domain comprising all three of: a light chain CDR1 of SEQ ID NO: 38, a light chain CDR2 of SEQ ID NO: 39 and a light chain CDR3 of SEQ ID NO: 40.

According to particular embodiments of said main embodiment, the combination is characterized in that the isolated antibody, or antigen-binding fragment thereof, directed against the viral Spike protein receptor binding-domain (RBD) of a Sarbecovirus, comprises:
- a heavy chain variable domain comprising all three of: a heavy chain CDR1 of SEQ ID NO: 41, a heavy chain CDR2 of SEQ ID NO: 42 and a heavy chain CDR3 of SEQ ID NO: 43; and a light chain variable domain comprising all three of: a light chain CDR1 of SEQ ID NO: 44, a light chain CDR2 of SEQ ID NO: 45 and a light chain CDR3 of SEQ ID NO: 46.

According to particular embodiments of said main embodiment, the combination is characterized in that the isolated antibody, or antigen-binding fragment thereof, directed against the viral Spike protein receptor binding-domain (RBD) of a Sarbecovirus, comprises:
- a heavy chain variable domain comprising all three of: a heavy chain CDR1 of SEQ ID NO: 47, a heavy chain CDR2 of SEQ ID NO: 48 and a heavy chain CDR3 of SEQ ID NO: 49; and a light chain variable domain comprising all three of: a light chain CDR1 of SEQ ID NO: 50, a light chain CDR2 of SEQ ID NO: 51 and a light chain CDR3 of SEQ ID NO: 52.

According to particular embodiments of said main embodiment, the combination is characterized in that the isolated antibody, or antigen-binding fragment thereof, directed against the viral Spike protein receptor binding-domain (RBD) of a Sarbecovirus, comprises:
- a heavy chain variable domain comprising all three of: a heavy chain CDR1 of SEQ ID NO: 53, a heavy chain CDR2 of SEQ ID NO: 54 and a heavy chain CDR3 of SEQ ID NO: 55; and a light chain variable domain comprising all three of: a light chain CDR1 of SEQ ID NO: 56, a light chain CDR2 of SEQ ID NO: 57 and a light chain CDR3 of SEQ ID NO: 58.

According to particular embodiments of said main embodiment, the combination is characterized in that the isolated antibody, or antigen-binding fragment thereof, directed against the viral Spike protein receptor binding-domain (RBD) of a Sarbecovirus, comprises:
- a heavy chain variable domain comprising all three of: a heavy chain CDR1 of SEQ ID NO: 59, a heavy chain CDR2 of SEQ ID NO:60 and a heavy chain CDR3 of SEQ ID NO: 61; and a light chain variable domain comprising all three of: a light chain CDR1 of SEQ ID NO: 62, a light chain CDR2 of SEQ ID NO: 63 and a light chain CDR3 of SEQ ID NO: 64.

According to particular embodiments of said main embodiment, the combination is characterized in that:
- the isolated antibody, or antigen-binding fragment thereof, directed against the viral Spike protein heptad repeat 2 (HR2) of a Sarbecovirus comprises: a heavy chain variable domain comprising all three of: a heavy chain CDR1 of SEQ ID NO: 1, a heavy chain CDR2 of SEQ ID NO: 2 and a heavy chain CDR3 of SEQ ID NO: 3; and a light chain variable domain comprising all three of: a light chain CDR1 of SEQ ID NO: 4, a light chain CDR2 of SEQ ID NO: 5 and a light chain CDR3 of SEQ ID NO: 6; and
- the isolated antibody, or antigen-binding fragment thereof, directed against the viral Spike protein receptor binding-domain (RBD) of a Sarbecovirus is as defined above.

According to exemplified embodiments of said main embodiment, the combination is characterized in that:
(i) the isolated antibody, or antigen-binding fragment thereof, directed against the viral Spike protein heptad repeat 2 (HR2) of a Sarbecovirus, comprises: a heavy chain variable domain comprising all three of: a heavy chain CDR1 of SEQ ID NO: 1, a heavy chain CDR2 of SEQ ID NO: 2 and a heavy chain CDR3 of SEQ ID NO: 3; and a light chain variable domain comprising all three of: a light chain CDR1 of SEQ ID NO: 4, a light chain CDR2 of SEQ ID NO: 5 and a light chain CDR3 of SEQ ID NO: 6; and
(ii) the isolated antibody, or antigen-binding fragment thereof, directed against the viral Spike protein receptor binding-domain (RBD) of a Sarbecovirus, is characterized in that it comprises: a heavy chain variable domain comprising all three of: a heavy chain CDR1 of SEQ ID NO: 41, a heavy chain CDR2 of SEQ ID NO: 42 and a heavy chain CDR3 of SEQ ID NO: 43; and a light chain variable domain comprising all three of: a light chain CDR1 of SEQ ID NO: 44, a light chain CDR2 of SEQ ID NO: 45 and a light chain CDR3 of SEQ ID NO: 46.

According to a **second main embodiment,** the invention relates to an isolated nucleic acid or expression vector or host cell coding for:
(i) an antibody, or antigen-binding fragment thereof, directed against the viral Spike protein heptad repeat 2 (HR2) of a Sarbecovirus, in particular of Severe Acute Respiratory Syndrome-related Coronavirus 2 (SARS-CoV-2); and
(ii) an antibody, or antigen-binding fragment thereof, directed against the viral Spike protein receptor binding-domain (RBD) of a Sarbecovirus, in particular of Severe Acute Respiratory Syndrome-related Coronavirus 2 (SARS-CoV-2);
characterized in that the antibody or antigen-binding fragment thereof directed against the viral Spike protein heptad repeat 2 (HR2) of a Sarbecovirus, binds specifically to a polypeptide comprising **SEQ ID NO: 26** within the polypeptide **SEQ ID NO: 65.**

According to a **third main embodiment,** the invention relates to a kit comprising:
- an antibody or antigen-binding fragment thereof, directed against the viral Spike protein heptad repeat 2 (HR2) of a Sarbecovirus, in particular of Severe Acute Respiratory Syndrome-related Coronavirus 2 (SARS-CoV-2), or nucleic acid or expression vector or host cell coding for the said antibody or antigen-binding fragment thereof; and
- an antibody or antigen-binding fragment thereof, directed against the viral Spike protein receptor binding-domain (RBD) of a Sarbecovirus, in particular of Severe Acute Respiratory Syndrome-related Coronavirus 2 (SARS-CoV-2), or nucleic acid or expression vector or host cell coding for the said antibody or antigen-binding fragment thereof;
characterized in that the isolated antibody or antigen-binding fragment thereof directed against the viral Spike protein heptad repeat 2 (HR2) of a Sarbecovirus, binds specifically to a polypeptide comprising **SEQ ID NO: 26** within the polypeptide **SEQ ID NO: 65.**

According to a **fourth main embodiment,** the invention relates to a pharmaceutical composition comprising the combination or nucleic acid or vector or host cell of the invention; and a pharmaceutically acceptable carrier.

According to particular embodiments, the combination, or the nucleic acid or vector or host cell, or the kit, or the pharmaceutical composition, is for use as a medicament.

According to particular embodiments, the combination, or the nucleic acid or vector or host cell, or the kit, or the pharmaceutical composition, is for use in a method for preventing and/or reducing the likelihood of occurrence and/or treating a Sarbecovirus infection and related disease, in particular a SARS-CoV-2 infection and associated COVID-19 disease.

According to a **fifth main embodiment,** the invention relates to an isolated antibody, or antigen-binding fragment thereof, directed against the viral Spike protein heptad repeat 2 (HR2) of a Sarbecovirus; for use in a method for preventing and/or reducing the likelihood of occurrence and/or treating a Sarbecovirus infection and related disease; characterized in that it comprises
- A heavy chain variable domain comprising all three of: a heavy chain CDR1 of SEQ ID NO: 1, a heavy chain CDR2 of SEQ ID NO: 2 and a heavy chain CDR3 of SEQ ID NO: 3; and a light chain variable domain comprising all three of: a light chain CDR1 of SEQ ID NO: 4, a light chain CDR2 of SEQ ID NO: 5 and a light chain CDR3 of SEQ ID NO: 6;
further characterized in that the isolated antibody or antigen-binding fragment thereof directed against the viral Spike protein heptad repeat 2 (HR2) of a Sarbecovirus, binds specifically to a polypeptide comprising **SEQ ID NO: 26** within the polypeptide **SEQ ID NO: 65.**

According to a **sixth main embodiment,** the invention relates to a nucleic acid or expression vector or host cell coding for an antibody, or antigen-binding fragment thereof, directed against the viral Spike protein heptad repeat 2 (HR2) of a Sarbecovirus; for use in a method for preventing and/or reducing the likelihood of occurrence and/or treating a Sarbecovirus infection and related disease;
characterized in that the antibody, or antigen-binding fragment thereof comprises a heavy chain variable domain comprising all three of: a heavy chain CDR1 of SEQ ID NO: 1, a heavy chain CDR2 of SEQ ID NO: 2 and a heavy chain CDR3 of SEQ ID NO: 3; and a light chain variable domain comprising all three of: a light chain CDR1 of SEQ ID NO: 4, a light chain CDR2 of SEQ ID NO: 5 and a light chain CDR3 of SEQ ID NO: 6; and
further characterized in that the antibody, or antigen-binding fragment thereof directed against the viral Spike protein heptad repeat 2 (HR2) of a Sarbecovirus, binds specifically to a polypeptide comprising **SEQ ID NO: 26** within the polypeptide **SEQ ID NO: 65.**

According to particular embodiments, the isolated antibody, or antigen-binding fragment thereof, or the nucleic acid or vector or host cell is for use in a method for preventing and/or reducing the likelihood of occurrence and/or treating a SARS-CoV-2 infection and associated COVID-19 disease.

According to particular embodiments, the isolated antibody, or antigen-binding fragment thereof, or the nucleic acid or vector or host cell for use according to the invention is characterized in that the antibody is a human neutralizing antibody.

According to particular embodiments, the isolated anti-HR2 antibody, or antigen-binding fragment thereof, or the nucleic acid or vector or host cell for use according to the invention is characterized in that the anti-HR2 antibody is a human neutralizing antibody

According to said particular embodiments, when referring to a combination of anti-RBD and anti-HR2 according to the present disclosure, in a non-exhaustive manner, this may thus encompass the following alternatives.

According to some alternatives, when referring to the combination of anti-RBD and anti-HR2 according to the present disclosure, the combination may comprise or consist of:
(i) the antibody, or antigen-binding fragment thereof, directed against the viral Spike protein heptad repeat 2 (HR2) of a Sarbecovirus; and
(ii) the antibody, or antigen-binding fragment thereof, directed against the viral Spike protein receptor binding-domain (RBD) of a Sarbecovirus which consists of a **human antibody or an antigen-binding fragment thereof.**

According to one of those alternatives, when referring to the combination of anti-RBD and anti-HR2 according to the present disclosure, the combination may comprise or consist of::
(i) the antibody, or antigen-binding fragment thereof, directed against the viral Spike protein heptad repeat 2 (HR2) of a Sarbecovirus which consists of a **human antibody or an antigen-binding fragment thereof;** and
(ii) the antibody, or antigen-binding fragment thereof, directed against the viral Spike protein receptor binding-domain (RBD) of a Sarbecovirus.

According to one of those alternatives, when referring to the combination of anti-RBD and anti-HR2 according to the present disclosure, the combination may comprise or consist of::
(i) the antibody, or antigen-binding fragment thereof, directed against the viral Spike protein heptad repeat 2 (HR2) of a Sarbecovirus which consists of a **human antibody or an antigen-binding fragment thereof;** and
(ii) the antibody, or antigen-binding fragment thereof, directed against the viral Spike protein receptor binding-domain (RBD) of a Sarbecovirus of a which consists of a **human antibody or an antigen-binding fragment thereof.**

According to some of those alternatives, when referring to the combination of anti-RBD and anti-HR2 according to the present disclosure, this may encompass embodiments in which the anti-RBD and the anti-HR2 are both recombinant antigen-binding fragments, for example human antigen-binding fragments, which may then be combined within a same binder, as a bi-specific or even multi-specific binder.

For example, the combination of anti-RBD and anti-HR2 according to the present disclosure may consist of a multi-specific binder comprising:
(i) the antigen-binding fragment directed against the viral Spike protein heptad repeat 2 (HR2) of a Sarbecovirus; and
(ii) the antigen-binding fragment directed against the viral Spike protein receptor binding-domain (RBD) of a Sarbecovirus.

Alternatively, the combination of anti-RBD and anti-HR2 according to the present disclosure may comprise, both, a multi-specific binder comprising either the anti-RBD or the anti-HR2, and a second distinct binder comprising the anti-HR2 or anti-RBD, respectively.

For example, the combination of anti-RBD and anti-HR2 according to the present disclosure may consist of:
(i) A **multi-specific binder** comprising the antigen-binding fragment directed against the viral Spike protein heptad repeat 2 (HR2) of a Sarbecovirus; and
(ii) the antibody, or antigen-binding fragment thereof, directed against the viral Spike protein receptor binding-domain (RBD).

For example, the combination of anti-RBD and anti-HR2 according to the present disclosure may consist of:
(i) the antibody, or antigen-binding fragment thereof, directed against the viral Spike protein heptad repeat 2 (HR2) of a Sarbecovirus; and
(ii) A **multi-specific binder** comprising the antigen-binding fragment thereof, directed against the viral Spike protein receptor binding-domain (RBD).

For example, the combination of anti-RBD and anti-HR2 according to the present disclosure may consist of:
(i) A **multi-specific binder** comprising the antigen-binding fragment thereof, directed against the viral Spike protein heptad repeat 2 (HR2) of a Sarbecovirus; and
(ii) A **multi-specific binder** comprising the antigen-binding fragment thereof, directed against the viral Spike protein receptor binding-domain (RBD).

According to particular embodiments, the isolated antibody, or antigen-binding fragment thereof, or the nucleic acid or vector or host cell for use according to the invention is characterized in that the antibody is **not** a single-domain antibody.

According to particular embodiments, the isolated anti-HR2 antibody, or antigen-binding fragment thereof, or the nucleic acid or vector or host cell for use according to the invention is characterized in that the anti-HR2 antibody is **not** a single-domain antibody.

According to some alternatives, when referring to the combination of anti-RBD and anti-HR2 according to the present disclosure, the combination may comprise or consist of:
(i) the antibody, or antigen-binding fragment thereof, directed against the viral Spike protein heptad repeat 2 (HR2) of a Sarbecovirus; and
(ii) the antibody, or antigen-binding fragment thereof, directed against the viral Spike protein receptor binding-domain (RBD) of a Sarbecovirus **which is not a single-domain antibody.**

According to one of those alternatives, when referring to the combination of anti-RBD and anti-HR2 according to the present disclosure, the combination may comprise or consist of:
(i) the antibody, or antigen-binding fragment thereof, directed against the viral Spike protein heptad repeat 2 (HR2) of a Sarbecovirus **which is not a single-domain antibody;** and
(ii) the antibody, or antigen-binding fragment thereof, directed against the viral Spike protein receptor binding-domain (RBD) of a Sarbecovirus.

According to one of those alternatives, when referring to the combination of anti-RBD and anti-HR2 according to the present disclosure, the combination may comprise or consist of:
(i) the antibody, or antigen-binding fragment thereof, directed against the viral Spike protein heptad repeat 2 (HR2) of a Sarbecovirus **which is not a single-domain antibody;** and
(ii) the antibody, or antigen-binding fragment thereof, directed against the viral Spike protein receptor binding-domain (RBD) of a Sarbecovirus.

According to one of those alternatives, when referring to the combination of anti-RBD and anti-HR2 according to the present disclosure, the combination may comprise or consist of:
(i) the antibody, or antigen-binding fragment thereof, directed against the viral Spike protein heptad repeat 2 (HR2) of a Sarbecovirus **which is not a single-domain antibody;** and
(ii) the antibody, or antigen-binding fragment thereof, directed against the viral Spike protein receptor binding-domain (RBD) of a Sarbecovirus **which is not a single-domain antibody.**

According to a **seventh main embodiment,** the invention relates to an *in vitro* method for detecting a Sarbecovirus in a sample, comprising a step of contacting said sample with:
- at least one antibody, or antigen-binding fragment thereof, directed against the viral Spike protein heptad repeat 2 (HR2) of a Sarbecovirus, in particular of Severe Acute Respiratory Syndrome-related Coronavirus 2 (SARS-CoV-2); and
- at least of one antibody, or antigen-binding fragment thereof, directed against the viral Spike protein receptor binding-domain (RBD) of a Sarbecovirus, in particular of Severe Acute Respiratory Syndrome-related Coronavirus 2 (SARS-CoV-2);
wherein the isolated antibody or antigen-binding fragment thereof directed against the viral Spike protein heptad repeat 2 (HR2) of a Sarbecovirus, binds specifically to a polypeptide comprising **SEQ ID NO: 26** within the polypeptide **SEQ ID NO: 65.**

The invention encompasses methods of making and using, as well as uses of the antibodies, nucleic acids, vectors, host cells according to the present disclosure, in particular for the detection, diagnosis, monitoring, prevention and treatment of a Sarbecovirus infection and associated disease, in particular a SARS-CoV-2 infection and associated disease (COVID-19).

Preferably the SARS-CoV-2 variant is chosen from the B.1.1.7 lineages and P.1 lineages and B.1.351 lineages and B.1.617 lineages and B.1.1.529 lineages and BA.1 sublineages and BA.2 sublineages and BA.2.75 sublineages and BA.4 sublineages and BA.5 sublineages and BF.7 sublineages and BQ.1.1 sublineages and XBB.1 sublineages and XBB.1.5 sublineages.

In some particular embodiments, the antibody or antigen-binding fragment, or combination thereof, according to the present disclosure binds to a plurality of Sarbecoviruses, including SARS-CoV-2 and SARS-CoV-1 , but does not cross-react with at least one human coronavirus selected from MERS-CoV, NL63-CoV, OC43-CoV, HKU1-CoV and 229E-CoV.

In some particular embodiments, the antibody or antigen-binding fragment, or combination thereof, according to the present disclosure binds specifically to the SarbecoviruseSARS-CoV-2 but does not cross-react with at least one human coronavirus selected from MERS-CoV, NL63-CoV, OC43-CoV, HKU1-CoV and 229E-CoV.

In some particular embodiments, the antibody or antigen-binding fragment, or combination thereof, according to the present disclosure binds specifically to the Sarbecoviruse SARS-CoV-1 but does not cross-react with at least one human coronavirus selected from MERS-CoV, NL63-CoV, OC43-CoV, HKU1-CoV and 229E-CoV.

In some more particular embodiments, the anti-RBD antibody or antigen-binding fragment according to the present disclosure binds to a plurality of Sarbecoviruses, including SARS-CoV-2 and SARS-CoV-1, but does not cross-react with at least one human coronavirus selected from MERS-CoV, NL63-CoV, OC43-CoV, HKU1-CoV and 229E-CoV.

In some other more particular embodiments, the anti-RBD antibody or antigen-binding fragment according to the present disclosure binds specifically to theSarbecoviruse SARS-CoV-2 but does not cross-react with at least one human coronavirus selected from MERS-CoV, NL63-CoV, OC43-CoV, HKU1-CoV and 229E-CoV.

In some other more particular embodiments, the anti-RBD antibody or antigen-binding fragment according to the present disclosure binds specifically to the Sarbecoviruse SARS-CoV-1 but does not cross-react with at least one human coronavirus selected from MERS-CoV, NL63-CoV, OC43-CoV, HKU1-CoV and 229E-CoV

In some more particular embodiments, the anti-HR2 antibody or antigen-binding fragment according to the present disclosure binds to a plurality of Sarbecoviruses, including SARS-CoV-2 and SARS-CoV-1, but does not cross-react with at least one human coronavirus selected from MERS-CoV, NL63-CoV, OC43-CoV, HKU1-CoV and 229E-CoV.

In some more particular embodiments, the anti-HR2 antibody or antigen-binding fragment according to the present disclosure binds to a plurality of Sarbecoviruses, including SARS-CoV-2, but does not cross-react with at least one human coronavirus selected from MERS-CoV, NL63-CoV, OC43-CoV, HKU1-CoV and 229E-CoV.

In some other particular embodiments, the anti-HR2 antibody or antigen-binding fragment according to the present disclosure binds to a plurality of Sarbecoviruses, including SARS-CoV-2, but does not bind to a SARS-CoV-2 variant comprising one or more mutations in the HR2 region selected from the group consisting of: a mutation D1199N.

In some more particular embodiments, the anti-HR2 antibody or antigen-binding fragment according to the present disclosure binds to a plurality of Sarbecoviruses, including SARS-CoV-1, but does not cross-react with at least one human coronavirus selected from MERS-CoV, NL63-CoV, OC43-CoV, HKU1-CoV and 229E-CoV.

In some other particular embodiments, the anti-HR2 antibody or antigen-binding fragment according to the present disclosure binds to a plurality of Sarbecoviruses, including SARS-CoV-1, but does not bind to a SARS-CoV-1 variant comprising one or more mutations in the HR2 region selected from the group consisting of: a mutation D1199N.

In some particular embodiments, the antibody or antigen-binding fragment or combination thereof, according to the present disclosure has normal levels of Antibody-dependent-cellular-cytotoxicity (ADCC) as compared to a control antibody; in particular the antibody has normal affinity to a CD16 (FcγRI) receptor as compared to a control antibody.

In some particular embodiments, the antibody or antigen-binding fragment or combination thereof, according to the present disclosure has modulated (i.e. low or decreased) levels of Antibody-dependent-cellular-cytotoxicity (ADCC) as compared to a control (positive or endogenous) antibody; in particular the antibody has modulated (i.e. low or decreased) affinity to a CD16 (FcγRI) receptor as compared to a control antibody.

In some more particular embodiments, the anti-RBD antibody or antigen-binding fragment according to the present disclosure has modulated (i.e. low or decreased) levels of Antibody-dependent-cellular-cytotoxicity (ADCC) as compared to a control (positive or endogenous) antibody; in particular the antibody has modulated (i.e. low or decreased) affinity to a CD16 (FcγRI) receptor as compared to a control antibody.

In some more particular embodiments, the anti-HR2 antibody or antigen-binding fragment according to the present disclosure has modulated (i.e. low or decreased) levels of Antibody-dependent-cellular-cytotoxicity (ADCC) as compared to a control (positive or endogenous) antibody; in particular the antibody has modulated (i.e. low or decreased) affinity to a CD16 (FcγRI) receptor as compared to a control antibody.

In some particular embodiments, the antibody or antigen-binding fragment or combination thereof, according to the present disclosure has low levels of Antibody-dependent-cellular-cytotoxicity (ADCC) as compared to a control antibody.

In some more particular embodiments, the anti-RBD antibody or antigen-binding fragment according to the present disclosure has low levels of Antibody-dependent-cellular-cytotoxicity (ADCC) as compared to a control antibody.

In some more particular embodiments, the anti-HR2 antibody or antigen-binding fragment according to the present disclosure has low levels of Antibody-dependent-cellular-cytotoxicity (ADCC) as compared to a control antibody.

In some particular embodiments, the antibody or antigen-binding fragment or combination thereof, according to the present disclosure has normal levels of Antibody-dependent-cellular-phagocytosis (ADCP) as compared to a control antibody; in particular the antibody has normal affinity to a CD32A (FcγRIIA) and CD64 (FcγRI) receptors as compared to a control antibody.

In some more particular embodiments, the anti-RBD antibody or antigen-binding fragment according to the present disclosure has normal levels of Antibody-dependent-cellular-phagocytosis (ADCP) as compared to a control antibody; in particular the antibody has normal affinity to a CD64 (FcγRI)receptor as compared to a control antibody.

In some more particular embodiments, the anti-HR2 antibody or antigen-binding fragment according to the present disclosure has normal levels of Antibody-dependent-cellular-phagocytosis (ADCP) as compared to a control antibody; in particular the antibody has normal affinity to a CD64 (FcγRI)receptor as compared to a control antibody.

In some particular embodiments, the antibody or antigen-binding fragment or combination thereof, according to the present disclosure has modulated (i.e. normal or improved) levels of Antibody-dependent-cellular-phagocytosis (ADCP) as compared to a control (positive or endogenous) antibody; in particular the antibody has modulated (i.e. normal or improved) affinity to a CD64 (FcγRI)receptor as compared to a control antibody.

In some more particular embodiments, the anti-HR2 antibody or antigen-binding fragment according to the present disclosure has modulated (i.e. normal or improved) levels of Antibody-dependent-cellular-phagocytosis (ADCP) as compared to a control (positive or endogenous) antibody; in particular the antibody has modulated (i.e. normal or improved) affinity to a CD64 (FcγRI) receptor as compared to a control antibody.

In some more particular embodiments, the anti-RBD antibody or antigen-binding fragment according to the present disclosure has modulated (i.e. normal or improved) levels of Antibody-dependent-cellular-phagocytosis (ADCP) as compared to a control (positive or endogenous) antibody; in particular the antibody has modulated (i.e. normal or improved) affinity to a CD64 (FcγRI) receptor as compared to a control antibody.

In some particular embodiments, the antibody or antigen-binding fragment, or combination thereof, according to the present disclosure has improved levels of Antibody-dependent-cellular-phagocytosis (ADCP) as compared to a control antibody.

In some more particular embodiments, the anti-RBD antibody or antigen-binding fragment according to the present disclosure has improved levels of Antibody-dependent-cellular-phagocytosis (ADCP) as compared to a control antibody.

In some more particular embodiments, the anti-HR2 antibody or antigen-binding fragment according to the present disclosure has improved levels of Antibody-dependent-cellular-phagocytosis (ADCP) as compared to a control antibody.

When determining the ADCP or ADCC activity of a given antibody, either anti-RBD or anti-HR2, the control (negative) antibody may be selected from mGO53 as the isotype control.

In some particular embodiments, the anti-RBD antibody or antigen-binding fragment thereof according to the present disclosure has no predicted reactivity to human proteins, no self-reactivity as compared to a control antibody, and/or no polyreactivity as compared to a control antibody. In some particular embodiments, the antibody or antigen-binding fragment according to the present disclosure is produced in a eukaryotic recombinant system.

In some particular embodiments, the antibody or antigen-binding fragment, or combination thereof, according to the present disclosure is produced in a eukaryotic recombinant system.

In some more particular embodiments, the anti-RBD antibody or antigen-binding fragment according to the present disclosure is produced in a eukaryotic recombinant system.

In some more particular embodiments, the anti-HR2 antibody or antigen-binding fragment according to the present disclosure is produced in a eukaryotic recombinant system.

In some particular embodiments, the antibody or antigen-binding fragment, or combination thereof, according to the present disclosure is produced in a prokaryotic recombinant system.

In some more particular embodiments, the anti-RBD antibody or antigen-binding fragment according to the present disclosure is produced in a prokaryotic recombinant system.

In some more particular embodiments, the anti-HR2 antibody or antigen-binding fragment according to the present disclosure is produced in a prokaryotic recombinant system.

In some particular embodiments, the antibody or antigen-binding fragment, or combination thereof, according to the present disclosure comprises a non-native human glycosylation pattern or a non-human glycosylation pattern.

In some more particular embodiments, the anti-RBD antibody or antigen-binding fragment, according to the present disclosure comprises a non-native human glycosylation pattern or a non-human glycosylation pattern.

In some more particular embodiments, the anti-HR2 antibody or antigen-binding fragment, according to the present disclosure comprises a non-native human glycosylation pattern or a non-human glycosylation pattern.

In some particular embodiments, the antibody or antigen-binding fragment, or combination thereof, according to the present disclosure is produced recombinantly and comprises a non-native human glycosylation pattern or a non-human glycosylation pattern.

In some more particular embodiments, the anti-RBD antibody or antigen-binding fragment, according to the present disclosure is produced recombinantly and comprises a non-native human glycosylation pattern or a non-human glycosylation pattern.

In some more particular embodiments, the anti-HR2 antibody or antigen-binding fragment, according to the present disclosure is produced recombinantly and comprises a non-native human glycosylation pattern or a non-human glycosylation pattern.

In some particular embodiments, the antibody or antigen-binding fragment, or combination thereof, according to the present disclosure is produced recombinantly and comprises a non-human glycosylation pattern.

In some more particular embodiments, the anti-RBD antibody or antigen-binding fragment, according to the present disclosure is produced recombinantly and comprises a non-human glycosylation pattern.

In some more particular embodiments, the anti-HR2 antibody or antigen-binding fragment, according to the present disclosure is produced recombinantly and comprises a non-human glycosylation pattern.

In some embodiments, the antibody according to the present disclosure or antigen-binding fragment thereof, or combination thereof, further comprises a detectable label.

In some more particular embodiments, the anti-RBD antibody according to the present disclosure or antigen-binding fragment thereof, further comprises a detectable label.

In some more particular embodiments, the anti-HR2 antibody according to the present disclosure or antigen-binding fragment thereof, further comprises a detectable label.

According to a **third main embodiment,** the invention relates to an isolated nucleic acid encoding an antibody or antigen-binding fragment according to the present disclosure; preferably comprising at least a nucleic acid sequence encoding the heavy and/or light chain of said antibody according to the present disclosure.

In some particular embodiment, the isolated nucleic acid encoding the anti-HR2 antibody or antigen-binding fragment according to the present disclosure comprises at least a nucleic acid sequence encoding the heavy chain of said antibody.

In some particular embodiment, the isolated nucleic acid encoding the anti-HR2 antibody or antigen-binding fragment according to the present disclosure comprises at least a nucleic acid sequence encoding the light chain of said antibody.

In some particular embodiment, the isolated nucleic acid encoding the anti-HR2 antibody or antigen-binding fragment according to the present disclosure comprises at least a nucleic acid sequence encoding the heavy chain of said antibody, and at least a nucleic acid sequence encoding the light chain of said antibody.

In some particular embodiments, the isolated nucleic acid according to the present disclosure is mRNA, preferably modified mRNA.

In some particular embodiments, the isolated nucleic acid according to the present disclosure is DNA.

Another aspect of the invention relates to an expression vector for the recombinant production of an antibody according to the present disclosure in a host cell, comprising at least one nucleic acid encoding said antibody according to the present disclosure.

In some particular embodiments, the isolated nucleic acid or expression vector according to the present disclosure comprises a nucleic acid sequence coding for a polypeptide having at least 90% identity with **SEQ ID NO: 15;** for example, a nucleic acid sequence coding for a polypeptide having at least 90%, 91%, 92%, 93%, 94%, 95%, 96%, 97%, 98%, or 99% identity with **SEQ ID NO:15.**

A nucleic acid coding for a variable light chain of an anti-HR2 of the disclosure is represented by **SEQ ID NO: 19.**

A nucleic acid coding for a variable heavy chain of an anti-HR2 of the disclosure is represented by **SEQ ID NO: 20.**

In some particular embodiments, the isolated nucleic acid or expression vector according to the present disclosure comprises a nucleic acid sequence having at least 90% identity with **SEQ ID NO: 19;** for example, a nucleic acid sequence having at least 90%, 91%, 92%, 93%, 94%, 95%, 96%, 97%, 98%, or 99% identity with **SEQ ID NO:19.**

In some particular embodiments, the isolated nucleic acid or expression vector according to the present disclosure comprises a nucleic acid sequence having at least 90% identity with **SEQ ID NO: 20;** for example, a nucleic acid sequence having at least 90%, 91%, 92%, 93%, 94%, 95%, 96%, 97%, 98%, or 99% identity with **SEQ ID NO:20.**

In some particular embodiments, the expression vector according to the present disclosure comprises a pair of nucleic acid sequences:
- At least one nucleic acid sequence coding for a polypeptide having at least 90% identity with **SEQ ID NO: 15;** for example, having at least 90%, 91%, 92%, 93%, 94%, 95%, 96%, 97%, 98%, or 99% identity with **SEQ ID NO:15;**
- At least one nucleic acid sequence coding for a polypeptide having at least 90% identity with **SEQ ID NO: 16;** for example, having at least 90%, 91%, 92%, 93%, 94%, 95%, 96%, 97%, 98%, or 99% identity with **SEQ ID NO:16.**

In some particular embodiments, the expression vector according to the present disclosure comprises a pair of nucleic acid sequences:
- At least one nucleic acid sequence having at least 90% identity with **SEQ ID NO: 19;** for example, having at least 90%, 91%, 92%, 93%, 94%, 95%, 96%, 97%, 98%, or 99% identity with **SEQ ID NO:19;**
- At least one nucleic acid sequence having at least 90% identity with **SEQ ID NO: 20;** for example, having at least 90%, 91%, 92%, 93%, 94%, 95%, 96%, 97%, 98%, or 99% identity with **SEQ ID NO:20.**

In some particular embodiments, the expression vector according to according to the present disclosure is contained in a bacteria strain chosen from Cv2.3132_plgH and Cv2.3132_plgL deposited under the terms of the Budapest Treaty at the Collection Nationale de Cultures de Microorganismes (CNCM) at the Institut Pasteur, 25 rue du Docteur Roux, 75724 Paris, FR, on March 31, 2023, under the number CNCM I-5943 and CNCM I-5944 respectively.

Another aspect relates to a host cell comprising an expression vector according to the present disclosure or a nucleic acid according to the present disclosure.

In some particular embodiments, the host cell according to the present disclosure is an antibody producing cell-line stably transformed with the expression vector.

In some particular embodiments, the host cell according to the present disclosure is a eukaryotic cell; preferably chosen from yeast, insect and mammalian cells.

Another aspect relates to a method of production of the antibody according to the present disclosure, comprising: (i) culturing the host cell of the present disclosure for expression of said antibody by the host cell; and optionally (ii) recovering the antibody; and (iii) purifying said antibody.

Another aspect relates to a pharmaceutical composition comprising the antibody, antigen-binding fragment thereof, nucleic acid or vector according to the present disclosure, and at least one of a pharmaceutically acceptable carrier, an adjuvant, and a preservative.

In some particular embodiments, the nucleic acid is mRNA, in particular modified mRNA; preferably formulated in a vesicle or particle, in particular a lipid nanoparticle (LNP).

In some particular embodiments, the pharmaceutical composition according to the present disclosure is for parenteral injection, infusion, local delivery, inhalation, or sustained delivery.

Another aspect relates to the antibody according to the present disclosure, antigen-binding fragment thereof, nucleic acid, vector, or pharmaceutical composition according to the present disclosure, for use as a medicament.

Another aspect relates to the pharmaceutical composition according to the present disclosure, for use in the prevention or treatment of a Sarbecovirus infection and associated disease, in particular of a SARS-CoV-2 infection and associated COVID-19 disease.

Another aspect relates to the use of a pharmaceutical composition according to the present disclosure for the manufacture of a medicament for the prevention or treatment of a Sarbecovirus infection and associated disease, in particular of a SARS-CoV-2 infection and associated COVID-19 disease.

Another aspect relates to a method for the detection of a Sarbecovirus, in particular a SARS-CoV-2, in a sample comprising: contacting said sample with an antibody according to the present disclosure or antigen-binding fragment thereof, and detecting the antigen-antibody complexes formed, thereby detecting the presence, absence or level of Sarbecovirus in the sample.

In some particular embodiments of the method according to the present disclosure, the sample is a biological sample from a subject suspected to be contaminated with a Sarbecovirus, in particular SARS-CoV-2, and the method is for the diagnosis of a Sarbecovirus infection and associated disease, in particular of a SARS-CoV-2 infection and associated COVID-19 disease.

In some particular embodiments of the method according to the present disclosure, the sample is a biological sample from a patient infected with a Sarbecovirus, in particular a COVID-19 patient before or during treatment of COVID-19 disease and the method is for the monitoring of treatment of COVID-19 disease.

Another aspect relates to a kit for the detection or diagnosis of a Sarbecovirus infection or contamination, or the monitoring of a treatment of a Sarbecovirus-related disease; in particular of a SARS-CoV-2 infection or contamination, or the monitoring of treatment of COVID-19 disease; comprising at least an antibody or combination of antibody according to the present disclosure, or antigen-binding fragment thereof, preferably further including a detectable label.

Another aspect relates to a method of reducing the risk of developing a Sarbecovirus-related disease in a subject, in particular a SARS-CoV-2-associated COVID-19 disease in a subject, comprising administering an effective amount of an antibody, an antigen-binding fragment thereof, or combination thereof, a nucleic acid or vector, or a pharmaceutical composition according to the present disclosure, to the subject.

In some particular embodiments of the method, the risk of hospitalization is reduced.

In some particular embodiments of the method, the risk of death is reduced.

Another aspect relates to a method of treating or preventing a Sarbecovirus-related disease in a subject, in particular a SARS-CoV-2-associated COVID-19 disease in a subject, comprising administering an effective amount of an antibody, an antigen-binding fragment thereof, or combination thereof, a nucleic acid or vector, or a pharmaceutical composition according to the present disclosure, to the subject.

Another aspect relates to a method of treating or preventing a Sarbecovirus-related disease in a subject, in particular a SARS-CoV-2-associated COVID-19 disease in a subject, comprising a step of:
a) detecting the Sarbecovirus in the subject, or a sample thereof;
b) treating the subject in which the Sarbecovirus is detected.

Hence, another aspect relates to a method of treating or preventing a Sarbecovirus-related disease in a subject, in particular a SARS-CoV-2-associated COVID-19 disease in a subject, comprising a step of:
a) contacting a sample from said subject with:
   - at least one antibody, or antigen-binding fragment thereof, directed against the viral Spike protein heptad repeat 2 (HR2) of a Sarbecovirus, in particular of Severe Acute Respiratory Syndrome-related Coronavirus 2 (SARS-CoV-2); and optionally
   - at least of one antibody, or antigen-binding fragment thereof, directed against the viral Spike protein receptor binding-domain (RBD) of a Sarbecovirus, in particular of Severe Acute Respiratory Syndrome-related Coronavirus 2 (SARS-CoV-2);
   wherein the isolated antibody or antigen-binding fragment thereof directed against the viral Spike protein heptad repeat 2 (HR2) of a Sarbecovirus, binds specifically to a polypeptide comprising **SEQ ID NO: 26** within the polypeptide **SEQ ID NO: 65;**
b) treating the patient in which the Sarbecovirus is detected.

In some particular embodiment, the step b) consists of administering an antibody directed against the viral Spike protein of the Sarbecovirus; more particularly an anti-RBD and/or an anti-HR2, and combinations thereof, according to the present disclosure.

In some particular embodiments of the method, the likelihood of developing severe disease is reduced by the treatment.

In some particular embodiments of the method, the likelihood of hospitalization is reduced by the treatment.

In some particular embodiments of the method, the subject is hospitalized.

Another aspect relates to a method of treating or preventing a Sarbecovirus-related disease in a subject, in particular a SARS-CoV-2-associated COVID-19 disease in a subject, comprising administering an effective amount of a combination of at least two antibodies, or antigen-binding fragments thereof, according to the present disclosure.

Another aspect relates to a method according to the present disclosure, wherein the subject is at risk of developing a Sarbecovirus-related disease, more particularly a subject with concurrent underlying conditions such as obesity, diabetes, cancer, under immunosuppressive therapy, primary immune deficiency or unresponsive to vaccines.

Another aspect relates to a method comprising administering an effective amount of an antibody, or antigen-binding fragment, or combination thereof or nucleic acid or vector, or pharmaceutical composition according to the present disclosure, for preventing and/or reducing the likelihood of occurrence of a Sarbecovirus infection; in particular a SARS-CoV-2 infection.

Another aspect relates to a method comprising administering an effective amount of an antibody, or antigen-binding fragment, or combination thereof, or nucleic acid or vector, or pharmaceutical composition according to the present disclosure, for preventing and/or reducing the likelihood of occurrence of a complication of a Sarbecovirus infection, in particular of a respiratory, nervous, gastrointestinal or cardiovascular complication of a Sarbecovirus infection; in particular of a SARS-CoV-2 infection.

Another particular aspect relates to a method comprising administering an effective amount of an antibody, or antigen-binding fragment, or combination thereof, or nucleic acid or vector, or pharmaceutical composition according to the present disclosure, for preventing and/or reducing the likelihood of occurrence of a severe acute respiratory complication of a Sarbecovirus infection; in particular of a SARS-CoV-2 infection.

Another aspect relates to a method comprising administering an effective amount of an antibody, or antigen-binding fragment, or combination thereof, or nucleic acid or vector, or pharmaceutical composition according to the present disclosure, for preventing and/or reducing the likelihood of occurrence of a Sarbecovirus infection in an individual, said individual being characterized in that
- the individual has not been administered a vaccine against the said Sarbecovirus infection, in particular of a SARS-CoV-2 infection; or
- the individual is not responding to the said vaccine; or
- the individual's level of antibodies directed against the Sarbecovirus infection, in particular of a SARS-CoV-2 infection, is at or below a protecting threshold level.

Another aspect relates to a method comprising administering an effective amount of an antibody, or antigen-binding fragment, or combination thereof, or nucleic acid or vector, or pharmaceutical composition according to the present disclosure, for improving an immune response against a Sarbecovirus virus; in particular of a SARS-CoV-2 infection.

Another particular aspect relates to a method comprising administering an effective amount of an antibody, or antigen-binding fragment, or combination thereof, or nucleic acid or vector, or pharmaceutical composition according to the present disclosure, for improving an immune response against a viral Spike protein of a Sarbecovirus virus; or a fragment thereof.

Another aspect relates to an antibody, or antigen-binding fragment, or nucleic acid or vector, or pharmaceutical composition according to the present disclosure, **in combination** with a vaccine against a Sarbecovirus infection, in particular of a SARS-CoV-2 infection; for use as a medicament.

Another aspect relates to an antibody, or antigen-binding fragment, or combination thereof, or nucleic acid or vector, or pharmaceutical composition according to the present disclosure, **in combination** with a further (e.g. third) antibody selected from the group of at least one of the following reference antibodies: Adintrevimab, Cilgavimab, Imdevimab, and Sotrovimab; for use as a medicament, in particular for the above-mentioned indications.

Another aspect relates to a use of an antibody, or antigen-binding fragment, or combination thereof, or nucleic acid or vector, or pharmaceutical composition according to the present disclosure, **in combination** with a further (e.g. third) antibody selected from the group of at least one of the following reference antibodies: Adintrevimab, Cilgavimab, Imdevimab, and Sotrovimab; for the preparation of a medicament.

The uses, methods, compositions and kits according to the present disclosure may be advantageously suitable for humans and non-human patients such as non-human mammals.

Another aspect relates to a medical device, comprising the pharmaceutical composition according to the present disclosure; preferably in a form suitable for administration by injection or inhalation.

### DETAILED DESCRIPTION OF THE INVENTION

The disclosure provides antibodies, including antigen-binding fragments thereof, and combinations of such antibodies or antigen-binding fragments against Sarbecovirus, and in particular SARS-CoV-2.

Surprisingly, the inventors have determined that antibodies directed against two epitopes of the Heptad repeat 2 (HR2) region of the Spike protein, and antigen-binding fragments thereof, can advantageously be combined with other antibodies or antigen-binding fragments directed against the RBD-domain of the same Spike protein.

Evidence is now shown, in a mouse model, anti-HR2 antibodies according to the present disclosure, leads to an enhanced neutralization effect toward a plurality of VOCs, with both therapeutic (after exposure) and prophylactic (before exposure) activity.

Advantageously, it is further found that the combination of anti-HR2 and anti-RBD domain antibodies, or antigen-binding fragments thereof, according to the present disclosure possess complementary and/or synergistic effects at certain concentrations.

Such anti-RBD domain antibodies, which are generally classified in the Art as class 1, 2, 3 or 4, may, in particular, include class 1 anti-RBD antibodies, such as those already isolated and characterized in WO2022228827A1**;** in a non-exhaustive manner, this may include at least one of the following anti-RBD antibodies or antigen-binding fragments thereof:
- a heavy chain variable domain comprising all three of: a heavy chain CDR1 of SEQ ID NO: 29, a heavy chain CDR2 of SEQ ID NO:30 and a heavy chain CDR3 of SEQ ID NO: 31; and a light chain variable domain comprising all three of: a light chain CDR1 of SEQ ID NO: 32, a light chain CDR2 of SEQ ID NO: 33 and a light chain CDR3 of SEQ ID NO: 34; or
- a heavy chain variable domain comprising all three of: a heavy chain CDR1 of SEQ ID NO: 35, a heavy chain CDR2 of SEQ ID NO:36 and a heavy chain CDR3 of SEQ ID NO: 37; and a light chain variable domain comprising all three of: a light chain CDR1 of SEQ ID NO: 38, a light chain CDR2 of SEQ ID NO: 39 and a light chain CDR3 of SEQ ID NO: 40; or
- a heavy chain variable domain comprising all three of: a heavy chain CDR1 of SEQ ID NO: 41, a heavy chain CDR2 of SEQ ID NO: 42 and a heavy chain CDR3 of SEQ ID NO: 43; and a light chain variable domain comprising all three of: a light chain CDR1 of SEQ ID NO: 44, a light chain CDR2 of SEQ ID NO: 45 and a light chain CDR3 of SEQ ID NO: 46; or
- a heavy chain variable domain comprising all three of: a heavy chain CDR1 of SEQ ID NO: 47, a heavy chain CDR2 of SEQ ID NO: 48 and a heavy chain CDR3 of SEQ ID NO: 49; and a light chain variable domain comprising all three of: a light chain CDR1 of SEQ ID NO: 50, a light chain CDR2 of SEQ ID NO: 51 and a light chain CDR3 of SEQ ID NO: 52; or
- a heavy chain variable domain comprising all three of: a heavy chain CDR1 of SEQ ID NO: 53, a heavy chain CDR2 of SEQ ID NO: 54 and a heavy chain CDR3 of SEQ ID NO: 55; and a light chain variable domain comprising all three of: a light chain CDR1 of SEQ ID NO: 56, a light chain CDR2 of SEQ ID NO: 57 and a light chain CDR3 of SEQ ID NO: 58; or
- a heavy chain variable domain comprising all three of: a heavy chain CDR1 of SEQ ID NO: 59, a heavy chain CDR2 of SEQ ID NO:60 and a heavy chain CDR3 of SEQ ID NO: 61; and a light chain variable domain comprising all three of: a light chain CDR1 of SEQ ID NO: 62, a light chain CDR2 of SEQ ID NO: 63 and a light chain CDR3 of SEQ ID NO: 64.

According to particular and exemplified embodiments of said main embodiment, the combination is characterized in that the (ii) isolated antibody, or antigen-binding fragment thereof, directed against the viral Spike protein receptor binding-domain (RBD) of a Sarbecovirus, comprises:
- heavy chain variable domain comprising all three of: a heavy chain CDR1 of SEQ ID NO: 29, a heavy chain CDR2 of SEQ ID NO:30 and a heavy chain CDR3 of SEQ ID NO: 31; and a light chain variable domain comprising all three of: a light chain CDR1 of SEQ ID NO: 32, a light chain CDR2 of SEQ ID NO: 33 and a light chain CDR3 of SEQ ID NO: 34.

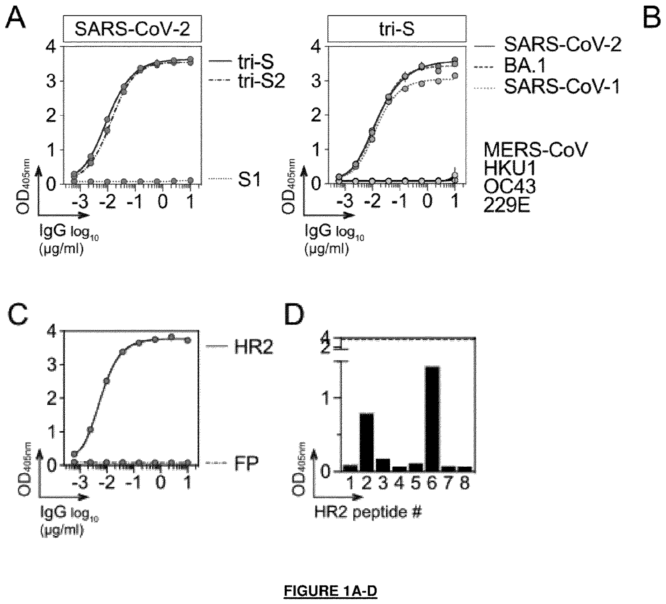

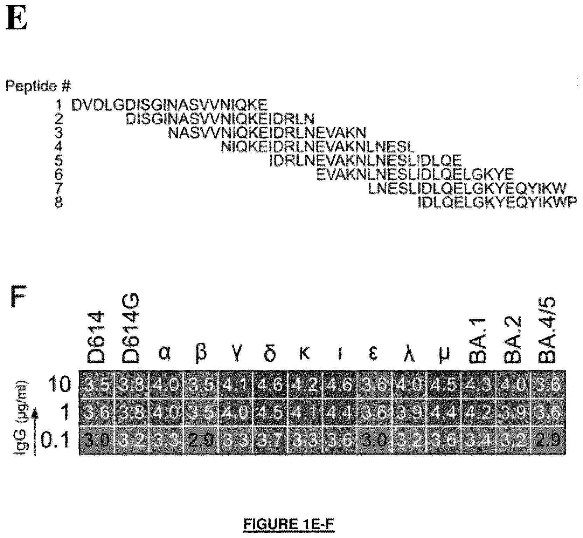

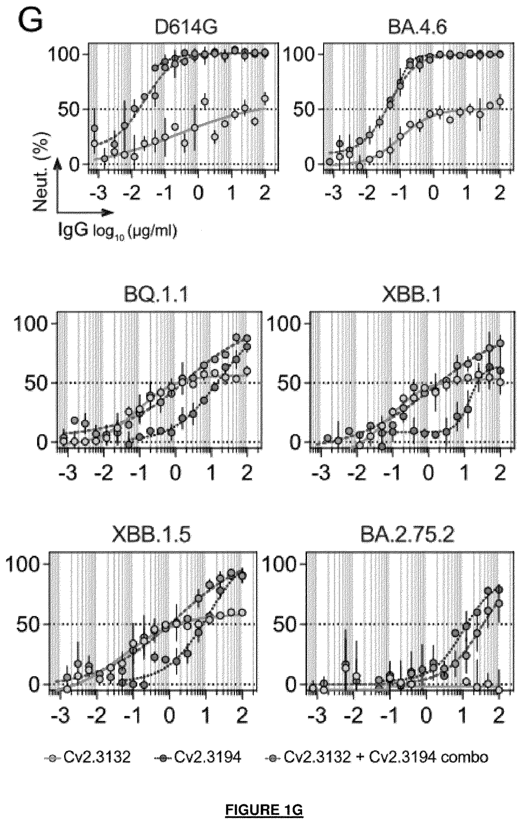

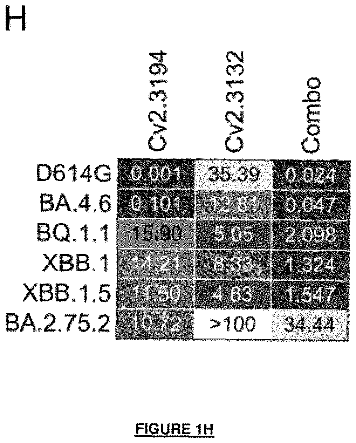

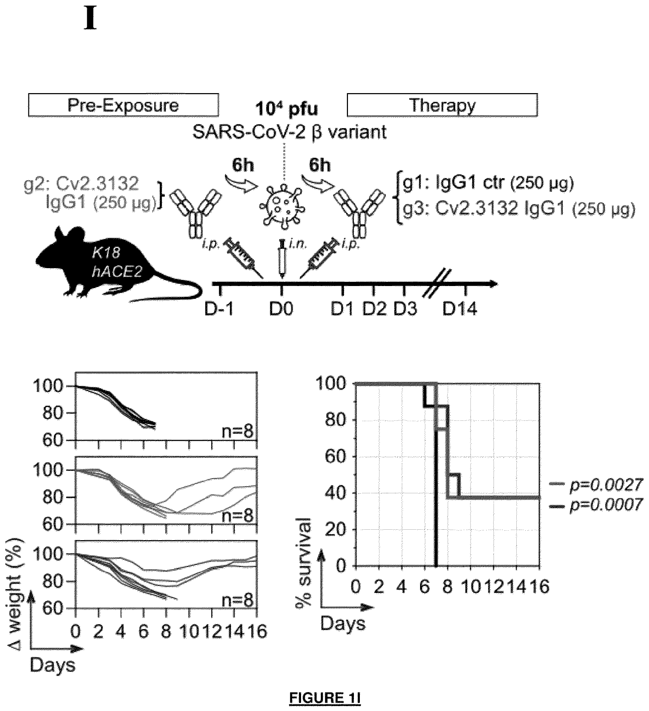

It is shown in **figure 1** that the combination of an anti-HR2 and of an anti-RBD leads to complementary and/or synergistic effects in a selection of VOCs, for which resistance to antibody neutralization has now been reported. Consequently, the inventors demonstrate, based on *in vivo* data, that the strategy consisting of targeting HR2 epitopes and RBD epitopes, for example in the context of an antibody therapy, is particularly convenient for reaching broad-spectrum neutralization in Sarbecoviruses.

The inventors have further characterized an anti-HR2 antibody, referred herein as Cv2.3132, with neutralization properties. Its mechanism of binding to HR2 led to the identification of a particular selection of epitopes, with therapeutic effects, within the HR2 region corresponding to **SEQ ID NO: 65,** and involving at least the polypeptide sequence **SEQ ID NO:** 26..

The anti-HR2 antibody referred herein as Cv2.3132 may be further characterized in that it comprises a variable heavy chain comprising sequence **SEQ ID NO: 15** and a variable light chain comprising sequence **SEQ ID NO: 16.**

The anti-HR2 antibody referred herein as Cv2.3132 may be further characterized in that it comprises a full length heavy chain comprising sequence **SEQ ID NO: 18** and a full length light chain comprising sequence **SEQ ID NO: 17.**

Without wishing to be bound by the theory, the inventors are of the opinion that the selected HR2 epitope is partly conformational, as it involves two non-continuous regions within **SEQ ID NO: 65,** with **SEQ ID NO: 26** being the most critical.

To their knowledge, the therapeutic effect associated to said HR2 epitope, both in the context of an isolated antibody or in combination with anti-RBD antibodies, has not previously been reported in the Art.

### Definitions

As used herein, the term "SARS-CoV-2 Spike (S) protein or glycoprotein" has its general meaning in the art and refers to a trimeric class I viral fusion protein (S trimer or tri-S) having the canonical sequence reported under accession number UniProtK P0DTC2. Based on structure predictions, signal peptide (SP) is from positions 1 to 12; ectodomain (extracellular domain) from positions 13 to 1213; transmembrane domain I from positions 1214 to 1234 and cytoplasmic domain from positions 1235 to 1273. S1 sub-unit is from positions 13 to 685, receptor-binding domain (RBD or RBD domain) from positions 319 to 541 and S2 sub-unit from positions 686 to 1273. However, the positions of the domains or sub-units may vary slightly (+1 to +15 and -1 to -15) relative to the indicated positions. For example, the signal peptide may be from positions 1 to 15, the ectodomain from positions 13 to 1208, S1 protein from positions 16 to 681, and RBD from positions 331 to 530 of the reference sequence.

Relative positions of each domain within the Spike protein or glycoprotein for other Sarbecoviruses, apart from SARS-CoV-2, may vary.

As used herein, the term "Heptad Repeat 2", or "HR2", in particular "SARS-CoV-2 HR2" refers to the polypeptide sequence ranging from position 1163 to position 1213 based on the canonical sequence reported under accession number UniProtK P0DTC2; which includes HR2 polypeptide sequences **SEQ ID NO: 21, SEQ ID NO: 22, SEQ ID NO: 23, SEQ ID NO: 24, SEQ ID NO: 25, SEQ ID NO: 26, SEQ ID NO: 27,** and **SEQ ID NO: 28,** and **SEQ ID NO:** 65; and more particularly **SEQ ID NO: 26.**

As used herein, the term "Sarbecovirus" is a *betacoronavirus* subgenus, which refers in particular to any betacoronavirus within lineage B, and includes lineage B viruses in clade la, clade lb, clade 2, and clade 3. Examples of clade la sarbecoviruses are SARS-CoV and Bat SARS-like coronavinis WIV1 (WIV1). Examples of clade lb sarbecoviruses are SARS-CoV-2, RatG13, Pangolin-Guanxi-(PANG/GX) and Pangolin-Guangdon-2019 (PANG/GD). Examples of clade 2 sarbecoviruses are Bat ZC45 (ZC45), Bat ZXC21 (ZXC21), YN2013, and RmYN02. Examples of clade 3 sarbecoviruses are BtkY72 and BGR2008. Hence, unless stated otherwise, an antibody or antigen-binding fragment thereof, or combination thereof, binds specifically to: a sarbecovirus of clade la (e.g., SARS-CoV, WIV1, or both); a sarbecovirus of clade lb (e.g., SARS-CoV-2, RatG13, Pangolin-Guanxi-2017 (PANG/GX), Pangolin-Guangdon-209, or any combination thereof); a sarbecovirus of clade 2; or a sarbecovirus of clade 3.

As used herein, SARS-CoV-2 refers to SARS-CoV-2 isolate Wuhan-Hu-1 and any isolate, strain, lineage, sublineage or variant thereof that is neutralized by the antibodies according to the invention. SARS-CoV-2 isolate Wuhan-Hu-1, which is used as SARS-CoV-2 reference is also referred to as BetaCoV_Wuhan_WIV04_2019 (EPI_ISL_402124) or BetaCoV_Wuhan_IVDC-HB-05_2019 EPI_ISL_402121. Non-limiting examples of SARS-CoV-2 variant or lineage which may be neutralized by the antibodies according to the present invention include SARS-CoV-2 variant comprising one or more mutations in the RBD selected from the group consisting of: G339D, G339H, R346T, F367V, S371F, S373P, S375F, T376A, D405N, R408S, K417N, K417T, N440K, K444T, V445P, G446S, L452R, N460K, S477N, T478K, E484A, E484K, E484Q, F486S, F486V, F486P, F490S, Q493R, G496S, Q498R, N501Y, Y505H, substitutions. The variant may comprise other mutations in the RBD, the Spike protein or any other viral proteins, which may not prevent neutralization by the antibodies according to the present disclosure.

The term "antibody" as used herein refers to immunoglobulin molecules and immunologically active portions of immunoglobulin molecules, i.e., molecules that contain an antigen binding site that immunospecifically binds an antigen. As such, the term antibody encompasses not only whole antibody molecules, but also antibody fragments as well as variants (including derivatives) of antibodies. The term may thus encompass conventional antibodies and non-conventional antibodies, such as single-domain domain, antibodies (SdAbs), for example of the V_{H}H type, and multispecific antibodies.

The term "antibody", as used herein and unless stated otherwise, may thus encompass whole antibody molecules, but also antigen-binding fragments thereof.

In natural antibodies of rodents and primates, two heavy chains are linked to each other by disulfide bonds, and each heavy chain is linked to a light chain by a disulfide bond. There are two types of light chains, lambda (λ) and kappa (κ). There are five main heavy chain classes (or isotypes) which determine the functional activity of an antibody molecule: IgM, IgD, IgG, IgA and IgE. In humans there are four subclasses of IgG: IgG1, IgG2, IgG3 and IgG4 (numbered in order of decreasing concentration in serum). IgA exists in two subclasses, IgA1 and IgA2. Both IgA1 and IgA2 have been found in external secretions (secretory IgA), where IgA2 is more prominent than in the blood (serum IgA). Each chain contains distinct sequence domains. In typical IgG antibodies, the light chain includes two domains, a variable domain (VL) and a constant domain (CL). The heavy chain includes four domains, a variable domain (VH) and three constant domains (CH1, CH2 and CH3, collectively referred to as CH). The variable regions of both light (VL) and heavy (VH) chains determine binding recognition and specificity to the antigen. The constant region domains of the light (CL) and heavy (CH) chains confer important biological properties such as antibody chain association, secretion, trans-placental mobility, complement binding, and binding to Fc receptors (FcR). Secretory IgA are polymeric: 2-4 IgA monomers are linked by two additional chains: the immunoglobulin joining (J) chain(s) and secretory component (SC). The J chain binds covalently to two IgA molecules through disulfide bonds between cysteine residues. The secretory component is a proteolytic cleavage product of the extracellular part of the polymeric immunoglobulin receptor (plgR) which binds to J-chain containing polymeric Ig. Polymeric IgA (mainly the secretory dimer) is produced by plasma cells in the lamina propria adjacent to mucosal surfaces. It binds to the polymeric immunoglobulin receptor on the basolateral surface of epithelial cells, and is taken up into the cell via endocytosis. The receptor-IgA complex passes through the cellular compartments before being secreted on the luminal surface of the epithelial cells, still attached to the receptor. Proteolysis of the receptor occurs, and the dimeric IgA molecule, along with a portion of the receptor known as the secretory component - known as sIgA, are free to diffuse throughout the lumen.

The Fv fragment is the N-terminal part of the Fab fragment of an immunoglobulin and consists of the variable portions of one light chain and one heavy chain. The specificity of the antibody resides in the structural complementarity between the antibody combining site and the antigenic determinant. Antibody combining sites are made up of residues that are primarily from the hypervariable or complementarity determining regions (CDRs). Occasionally, residues from non-hypervariable or framework regions (FR) can participate in the antibody binding site or influence the overall domain structure and hence the combining site. Complementarity Determining Regions or CDRs refer to amino acid sequences which together define the binding affinity and specificity of the natural Fv region of a native immunoglobulin binding site. The light and heavy chains of an immunoglobulin each have three CDRs, designated L-CDR1, L-CDR2, L- CDR3 and H-CDR1, H-CDR2, H-CDR3, respectively. An antigen-binding site, therefore, typically includes six CDRs, comprising the CDRs set from each of a heavy and a light chain V region. Framework Regions (FRs) refer to amino acid sequences interposed between CDRs. Accordingly, the variable regions of the light and heavy chains typically comprise 4 framework regions and 3 CDRs of the following sequence: FR1-CDR1-FR2-CDR2-FR3-CDR3-FR4.

The residues in antibody variable domains are conventionally numbered according to a system devised by Kabat et al. This system is set forth in Kabat et al., 1987, in Sequences of Proteins of Immunological Interest, US Department of Health and Human Services, NIH, USA (Kabat et al., 1992, hereafter "Kabat et al."). This numbering system is used in the present specification. The Kabat residue designations do not always correspond directly with the linear numbering of the amino acid residues in SEQ ID sequences. The actual linear amino acid sequence may contain fewer or additional amino acids than in the strict Kabat numbering corresponding to a shortening of, or insertion into, a structural component, whether framework or complementarity determining region (CDR), of the basic variable domain structure. The correct Kabat numbering of residues may be determined for a given antibody by alignment of residues of homology in the sequence of the antibody with a "standard" Kabat numbered sequence. The CDRs of the heavy chain variable domain are located at residues 31-35 (H-CDR1), residues 50-65 (H-CDR2) and residues 95-102 (H-CDR3) according to the Kabat numbering system. The CDRs of the light chain variable domain are located at residues 24-34 (L-CDR1), residues 50-56 (L-CDR2) and residues 89-97 (L-CDR3) according to the Kabat numbering system. The predicted CDRs of some anti-SARS-CoV-2 antibodies, such as Cv2.1169, Cv2.5213, Cv2.3235, Cv2.1353 and Cv2.3194 are described herein.

The term "monoclonal antibody" as used herein refers to a preparation of antibody molecules of single specificity. A monoclonal antibody displays a single binding specificity and affinity for a particular epitope. Accordingly, the term "human monoclonal antibody" refers to an antibody displaying a single binding specificity which has variable and constant regions derived from or based on human germline immunoglobulin sequences or derived from completely synthetic sequences. The method of preparing the monoclonal antibody is not relevant for the binding specificity.

As used herein, the term "recombinant antibody" refers to antibodies which are produced, expressed, generated or isolated by recombinant means, such as antibodies which are expressed using a recombinant expression vector transfected into a host cell; antibodies isolated from a recombinant combinatorial antibody library; antibodies isolated from an animal (e.g. a mouse) which is transgenic due to human immunoglobulin genes; or antibodies which are produced, expressed, generated or isolated in any other way in which particular immunoglobulin gene sequences (such as human immunoglobulin gene sequences) are assembled with other DNA sequences. Recombinant antibodies include, for example, chimeric and humanized antibodies. In some embodiments a recombinant human antibody of this invention has the same amino acid sequence as a naturally-occurring human antibody but differs structurally from the naturally occurring human antibody. For example, in some embodiments the glycosylation pattern is different as a result of the recombinant production of the recombinant human antibody. In some embodiments the recombinant human antibody is chemically modified by addition or subtraction of at least one covalent chemical bond relative to the structure of the human antibody that occurs naturally in humans.

As used herein, the term "non-native human glycosylation pattern" refers to a glycosylation pattern (i.e. of an antibody according to the present disclosure) which is characterized in that it is produced in human cells (i.e. *in vitro* production; for example *in vitro* production in HEK cells), and which may or may not correspond to the native glycosylation pattern of a reference human antibody.

As used herein, the term "non-human glycosylation pattern" refers to a glycosylation pattern which is characterized in that it is produced in non-human cells (i.e. *in vitro* production in CHO cells).

The term "antigen-binding fragment" of an antibody (or simply "antibody fragment"), as used herein, refers to full length or one or more fragments of an antibody that retain the ability to specifically bind to an antigen (e.g., Spike glycoprotein of SARS-CoV-2). It has been shown that the antigen-binding function of an antibody can be performed by fragments of a full-length antibody. Examples of binding fragments encompassed within the term "antigen-binding fragment" of an antibody include a Fab fragment, a monovalent fragment consisting of the VL, VH, CL and CH1 domains; a F(ab')2 fragment, a bivalent fragment comprising two Fab fragments linked by a disulfide bridge at the hinge region; a Fd fragment consisting of the VH and CH1 domains; a Fv fragment consisting of the VL and VH domains of a single arm of an antibody; a dAb fragment (Ward et al., 1989 Nature 341:544-546), which consists of a VH domain, or any fusion proteins comprising such antigen-binding fragments. Furthermore, although the two domains of the Fv fragment, VL and VH, are coded for by separate genes, they can be joined, using recombinant methods, by a synthetic linker that enables them to be made as a single chain protein in which the VL and VH regions pair to form monovalent molecules (known as single chain Fv (scFv); see e.g., Bird et al., 1988 Science 242:423-426; and Huston et al., 1988 Proc. Natl. Acad. Sci. 85:5879-5883). Such single chain antibodies are also intended to be encompassed within the term "antigen-binding fragment" of an antibody. These antibody fragments are obtained using conventional techniques known to those of skill in the art, and the fragments are screened for utility in the same manner as are intact antibodies.

The expression "variable domain" or "variable region" of an antibody heavy or light chain are used interchangeably as the variable region of an antibody consists of a variable domain.

The phrases "an antibody recognizing an antigen (X)", "an antibody having specificity for an antigen (X)", "an anti-X antibody", "an antibody against X", and an "antibody directed against" are used interchangeably herein with the term "an antibody which binds specifically to an antigen (X)".

As used herein, "antibody" or "nucleic acid" refers to an isolated antibody or nucleic acid.

As used herein a "class 1 anti-SARS-CoV2 Spike protein antibody" refers to a neutralizing antibody which specifically binds to the viral Spike protein receptor-binding domain (RBD) in the 'up' conformation.

As used herein a "class 2 anti-SARS-CoV2 Spike protein antibody" refers to a neutralizing antibody which may specifically bind to, both, the viral Spike protein receptor-binding domain (RBD) in the 'up' conformation and the viral Spike protein receptor-binding domain (RBD) in the 'down' conformation.

The "up" conformation of the RBD corresponds to the RBD conformation exposing the receptor-binding site to the peptidase domain (PD) of the angiotensin-converting enzyme 2 (ACE2). The "down" conformation of the RBD corresponds to the closed RBD conformation for which the ACE2 receptor-binding site is not accessible.

As used herein a "class 3 anti-SARS-CoV2 Spike protein antibody" refers to a neutralizing antibody which binds outside the ACE2-binding site of the RBD. The classification into class 2 or class 3 anti-SARS-CoV2 Spike protein antibodies corresponds to the Barnes classification which is developed in Barnes et al. ("SARS-CoV-2 neutralizing antibody structures inform therapeutic strategies"; Nature; 588, 682-687 (2020)).

The present disclosure encompasses the therapeutic use of both the antibody or antigen-binding fragment, or combination thereof, according to the present disclosure (antibody therapy) and a nucleic acid or vector encoding said antibody or antigen-binding fragment, in particular mRNA such as modified mRNA (nucleic acid therapy).

The term "Kassoc" or "Ka", as used herein, is intended to refer to the association rate of a particular antibody-antigen interaction, whereas the term "Kdis" or "Kd," as used herein, is intended to refer to the dissociation rate of a particular antibody-antigen interaction.

The term "KD", as used herein, is intended to refer to the dissociation constant, which is obtained from the ratio of Kd to Ka (i.e. Kd/Ka) and is expressed as a molar concentration (M). KD values for antibodies are determined by Surface plasmon resonance using Biacore^{®} system (Biacore assay).

As used herein, the term "specificity" refers to the ability of an antibody, or antigen-binding fragment thereof, to detectably bind an epitope presented on an antigen, while not detectably binding to (i.e., cross-reacting with) other epitopes.

According to some embodiments, an antibody (such as an anti-RBD antibody of the present disclosure) specifically binds to its target when it has a KD of 1 µM or less for its target in a Biacore assay. Antibodies are covalently coupled to CM5 sensor chips (Biacore) using amino-coupling kit (Biacore) according to the manufacturer's procedure. Recombinant ACE2 ectodomain may also be coupled to the sensor chip in the same conditions, for comparison. All analyses are performed using HBS-EP buffer (10 mM HEPES pH 7.2; 150 mM NaCl; 3 mM EDTA, and 0.005 % Tween 20). The flow rate of buffer during all real-time interaction measurements is set at 30 µl/min. All interactions are performed at temperature of 25 °C. SARS CoV-2 tri-S and S1 proteins are serially diluted (two-fold step) in HBS-EP in the range of 40 - 0.156 nM. Same range of concentrations is used for RBD with exception of low affinity interactions where the concentration range 1280 - 10 nM was applied. The association and dissociation phases of the binding of viral proteins to the immobilized antibodies and ACE2 are monitored for 3 and 4 minutes, respectively. The binding of the proteins to reference channel containing carboxymethylated dextran only is used as negative control and is subtracted from the binding during data processing. The evaluation kinetic parameters of the studied interactions are performed by using BIAevaluation version 4.1.1 Software (Biacore).

Specificity can further be exhibited by, e.g., an about 10:1, about 20:1, about 50:1, about 100:1, 10.000:1 or greater ratio of affinity/avidity in binding to the specific antigen versus nonspecific binding to other irrelevant antigen. For example, specificity demonstrated experimentally for at least the HR2 or the RBD and one non-specific antigen means that the antibody is specific to the said antigen. Experimental demonstration of specificity, for example toward an HR2 epitope of the present disclosure, may also be evidenced by ELISA for a given anti-HR2 antibody, such as a Cv2.3132 IgG, to HR2 peptides as previously described in Planchais et al. ("Potent human broadly SARS-CoV-2-neutralizing IgA and IgG antibodies effective against Omicron BA.1 and BA.2"; J. Exp. Med. 2022; 219(7):e20220638).

The term "affinity", as used herein, means the strength of the binding of an antibody to an epitope; for example a RBD epitope or an HR2 epitope.

As used herein, the term "Avidity" refers to an informative measure of the overall stability or strength of the antibody-antigen complex. It is controlled by three major factors: antibody epitope affinity; the valence of both the antigen and antibody; and the structural arrangement of the interacting parts. Ultimately these factors define the specificity of the antibody, that is, the likelihood that the particular antibody is binding to a precise antigen epitope.

As used herein, the term "neutralizing antibody" refers to an antibody that inhibits virus infection, in particular that inhibits or blocks virus entry into host cells. The neutralizing activity of the antibody is measured by SARS-CoV-2 S-Fuse Assay, as disclosed in Planas et al. ("Resistance of Omicron subvariants BA.2.75.2, BA.4.6, and BQ.1.1 to neutralizing antibodies"; Nat. Commun. 2023; 14(1):824). SARS-CoV-2 virus (Multiplicity of infection (MOI) of 0.1) is incubated with recombinant monoclonal IgA or IgG antibodies at 10 µg/ml or 5 µg/ml, and consecutive 1:4 dilutions in culture medium for 30 min at room temperature and added to S-Fuse cell culture (U2OS-ACE2 GFP1-10 and U2OS-ACE2 GFP 11; ratio 1:1; 8 × 10³ per well). After 18h of incubation, cells are fixed and nuclei stained. The area displaying GFP expression and the number of nuclei are quantified by confocal microscopy. The percentage neutralization is calculated from the GFP-positive area as follows: 100 × (1 - (value with IgA/IgG - value in "non-infected") / (value in "no IgA/IgG" - value in "non-infected")). The neutralizing activity of each isotype is expressed as the half maximal effective concentration (EC₅₀). EC₅₀ values (ng/ml) are calculated based on a reconstructed curve of the percentage neutralization at the various concentrations indicated. The neutralization activity may thus be modulated depending, for a particular VOC, depending on whether the given antibody is administered in an isolated form or in combination with a second antibody. For example, the neutralization activity for an isolated anti-RBD antibody may be decreased when administered in combination with an anti-HR2 antibody of the disclosure by 10-fold or 100-fold. According to some embodiments, a neutralizing antibody, or neutralizating combination of antibodies, may have an EC₅₀ of less than 10000 ng/mL, for example less than 9000 ng/mL, less than 8000 ng/mL, less than 7000 ng/mL, less than 6000 ng/mL, less than 5000 ng/mL, less than 4000 ng/mL, less than 3000 ng/mL, less than 2000 ng/mL, or 1000 ng/mL and/or neutralizes at least 50%, 60%, 70%, 80% or 90 % of SARS-CoV-2 in SARS-CoV-2 S-Fuse Assay.

As used herein, the term "ADCC" or "antibody dependent cell cytotoxicity" and "CDC" or "Complement-dependent-cytotoxicity" activity refers to cell depleting activity. ADCC and CDC activities can be measured by standard methods that are well-known in the art and disclosed in the examples The ADCC activity of the antibody of the present disclosure is quantified using the ADCC Reporter Bioassay (Promega). Raji-Spike cells (5×10⁴) are co-cultured with Jurkat-CD16-NFAT-rLuc cells (5×10⁴) in presence or absence of SARS-CoV2 S-specific or control mGO53 IgG antibody (3) at 10 µg/ml or 50 µg/ml and 10 consecutive 1:2 dilutions in PBS. Luciferase activity is measured after 18 h of incubation. ADCC is measured as the fold induction of Luciferase activity compared to the control antibody. A low ADCC activity is less than 2-fold higher compared to control antibody. The CDC activity of the antibody of the present disclosure is quantified using SARS-CoV-2 Spike-expressing Raji cells as previously described (**10**). Raji-Spike cells (5×10⁴) are cultivated in the presence of 50% normal (NHS) or heat-inactivated (HIHS) human serum and with or without recombinant IgG antibodies (at 10 µg/ml or 50 µg/ml and 10 consecutive 1:2 dilutions in PBS). After 24h, cells are washed with PBS and the live/dead fixable aqua dead cell marker (1:1,000 in PBS; Life Technologies) is added for 30 min at 4°C before fixation. Cells are analysed by fluorescence microscopy. CDC is calculated using the following formula: 100 × (% of dead cells with serum - % of dead cells without serum) / (100 - % of dead cells without serum). A low CDC activity is less than 3 %.

As used herein "preventing" a Sarbecovirus infection and/or associated/related disease, for example a SARS-CoV-2 infection and/or associated disease, means reducing the risk of said viral infection and/or associated disease.

It is further contemplated that antibodies or antigen-binding fragment thereof, and combinations thereof, may be further screened or optimized for their neutralizing properties as above defined. In particular, it is contemplated that monoclonal antibodies or antigen-binding fragment thereof may have 1, 2, 3, 4, 5, 6, or more alterations in the amino acid sequence of 1, 2, 3, 4, 5, or 6 CDRs of monoclonal antibodies provided herein, in particular in the CDR of SEQ ID NO: 1-6 for anti-HR2 antibodies or antigen-binding fragment thereof, and in the CDR of SEQ ID NO 29-64 for anti-RBD antibodies or antigen-binding fragment thereof. It is contemplated that the amino acid in position 1, 2, 3, 4, 5, 6, 7, 8, 9, or 10 of CDR1, CDR2, CDR3, CDR4, CDR5, or CDR6 of the VJ or VDJ region of the light or heavy variable region of antibodies may have an insertion, deletion, or substitution with a conserved or non-conserved amino acid. Such amino acids that can either be substituted or constitute the substitution are disclosed below. In some particular embodiments, the monoclonal antibodies or antigen-binding fragment have 1 or 2 conservative substitutions in the amino acid sequence of 1, 2, 3, 4, 5, or 6 CDRs of monoclonal antibodies provided herein.

In some embodiments, the amino acid differences are conservative substitutions, i.e., substitutions of one amino acid with another having similar chemical or physical properties (size, charge or polarity), which substitution generally does not adversely affect the biochemical, biophysical and/or biological properties of the antibody. In particular, the substitution does not disrupt the interaction of the antibody with the spike glycoprotein antigen and neutralizing properties. Said conservative substitution(s) are advantageously chosen within one of the following five groups: Group 1-small aliphatic, non-polar or slightly polar residues (A, S, T, P, G); Group 2-polar, negatively charged residues and their amides (D, N, E, Q); Group 3-polar, positively charged residues (H, R, K); Group 4-large aliphatic, nonpolar residues (M, L, I, V, C); and Group 5-large, aromatic residues (F, Y, W).

Neutralizing antibodies or antigen-binding fragments thereof according to the present disclosure defined by their CDR domains may comprise framework regions FR1, FR2, FR3 and FR4.

In a particular embodiment of the disclosure, the variable regions of the antibody as described above may be associated with antibody constant regions, like IgA, IgM, IgE, IgD or IgG such as Igg1, IgG2, IgG3, IgG4. Said variable regions of the antibody is preferably associated with IgG or IgA constant region; preferably IgG1 or IgA (IgA1, IgA2) constant regions. These constant regions may be further mutated or modified, by methods known in the art, in particular for modifying their binding capability towards Fc receptor or enhancing antibody half-life. The antibody comprising IgA constant region may further comprise a J chain and/or a secretory component to generate a polymeric or secretory IgA.

As used herein, the term "IgG Fc region" is used to define the C-terminal region of an immunoglobulin heavy chain, including native sequence Fc region and variant Fc regions. The human IgG heavy chain Fc region is generally defined as comprising the amino acid residue from position C226 or from P230 to the carboxyl-terminus of the IgG antibody. The numbering of residues in the Fc region is that of the EU index of Kabat. The C-terminal lysine (residue K447) of the Fc region may be removed, for example, during production or purification of the antibody. Accordingly, a composition of antibodies of the disclosure may comprise antibody populations with all K447 residues removed, antibody populations with no K447 residues removed, and antibody populations having a mixture of antibodies with and without the K447 residue.

In a particular embodiment, the antibody, or combination thereof (i.e. of anti-HR2 and anti-RBD) according to the present disclosure is - or comprises - a silent antibody. As used herein, the term "silent" antibody refers to an antibody that exhibits no or low ADCC activity. Silenced effector functions can be obtained by mutation in the Fc region of the antibodies and have been described in the Art: Strohl 2009 (LALA & N297A); Baudino 2008, D265A (Baudino et al., J. Immunol. 181 (2008): 6664-69, Strohl, CO Biotechnology 20 (2009): 685-91). Examples of silent Fc IgG1 antibodies comprise N297A or L234A and L235A mutations in the IgG1 Fc amino acid sequence.

In another particular embodiment of any of the antibodies or antigen-binding fragments described herein, and combinations thereof, the variant human Fc constant region comprises the M428L and N434S substitutions (LS) in EU index of Kabat to enhance antibody half-life. In some embodiments, the antibody of the present disclosure does not comprise an Fc domain capable of substantially binding to a FcgRIIIA (CD16) polypeptide. In some embodiments, the antibody of the present disclosure lacks an Fc domain (e.g. lacks a CH2 and/or CH3 domain).

Other mutations are G236A/A330L/I332E, herein termed "GAALIE which improves antiviral efficacy against pathogens (Bournazos et al., Nature, 2020, 588, 485-490). The M428L and N434S substitutions (LS) are advantageously combined with G236A/A330L/1332E.

Another modification of the antibodies herein that is contemplated by the present disclosure is pegylation or hesylation or related technologies. An antibody can be pegylated to, for example, increase the biological (e.g., serum) half-life of the antibody. To pegylate an antibody, the antibody, or fragment thereof, typically is reacting with polyethylene glycol (PEG), such as a reactive ester or aldehyde derivative of PEG, under conditions in which one or more PEG groups become attached to the antibody or antibody fragment. The pegylation can be carried out by an acylation reaction or an alkylation reaction with a reactive PEG molecule (or an analogous reactive water-soluble polymer). As used herein, the term "polyethylene glycol" is intended to encompass any of the forms of PEG that have been used to derivatize other proteins, such as mono (C1-C10) alkoxy- or aryloxy-polyethylene glycol or polyethylene glycol-maleimide. In certain embodiments, the antibody to be pegylated is an aglycosylated antibody. Methods for pegylating proteins are known in the art and can be applied to the antibodies of the disclosure. See for example, EP 0 154 316 by Nishimura et al. and EP 0 401 384 by Ishikawa et al.

Another modification of the antibodies that is herein contemplated is a conjugate or a fusion protein. The antibody or antigen-binding fragment thereof may be fused to another protein moiety of interest or conjugated to an agent of interest. The agent may be for example a therapeutic agent; a label for antibody detection or a protein which increases the half-life of the antibody. In some embodiments, at least the antigen-binding region of the antibody of the present disclosure is fused to a serum protein, such as human serum albumin or a fragment thereof to increase half-life of the resulting molecule. In other embodiments, the antibody or antigen binding fragment according to the present disclosure further comprises a detectable label. Preferred labels include fluorophores such as umbelliferone, fluorescein, fluorescein isothiocyanate (FITC), rhodamine, tetramethyl rhodamine, eosin, green fluorescent protein, erythrosin, coumarin, methyl coumarin, pyrene, malachite green, stilbene, lucifer yellow, Cascade Blue, Texas Red, dichlorotriazinylamine fluorescein, dansyl chloride, phycoerythrin, fluorescent lanthanide complexes such as those including Europium and Terbium, cyanine dye family members, such as Cy3 and Cy5, molecular beacons and fluorescent derivatives thereof, a chromophore label; a luminescent label such as luminol; a radioactive label such as ¹⁴C, ¹²³I, ¹²⁴I, ¹²⁵I, ³²P, ³³P, ³⁵S, or ³H and others; an affinity-ligand label, such as streptavidin/biotin, avidin/biotin or anti-isotype antibody; an enzyme label, such as alkaline phosphatase, horseradish peroxidase, luciferase, β galactosidase or acetylcholinesterase; an enzyme cofactor label; a hapten conjugate label, such as digoxigenin or dinitrophenyl; a Raman signal generating label; a magnetic label; a spin label; an epitope label, such as the FLAG or HA epitope; a heavy atom label; a nanoparticle label, an electrochemical label; a light scattering or plasmon resonant materials such as gold or silver particles or quantum dots; a spherical shell label; semiconductor nanocrystal label; as well as others known in the art, wherein the label can allow visualization with or without a secondary detection molecule.

In some embodiments, said antibody is polymeric. The polymeric antibody comprises or consists of Ig polymers. The polymeric antibody is preferably a polymeric monoclonal antibody derived from a monoclonal antibody as defined above. The Ig polymers comprise or consist of dimers. The polymeric antibody usually comprises immunoglobulin joining (J) chain(s) in addition to Ig molecules. The J chain is a 137 amino acid polypeptide expressed by plasma or myeloma cells which regulate Ig polymer formation by binding covalently to two Ig molecules through disulfide bonds between cysteine residues. In particular, dimeric antibodies are formed by two monomeric Ig molecules, which covalently bind to a J chain. In a preferred embodiment, said antibody is a polymeric IgA, preferably a polymeric IgA monoclonal antibody derived from a monoclonal antibody as defined above.

In some embodiments the antibody is a secretory antibody. A secretory antibody can be transported across epithelial cells to the luminal surface of serosal tissues. The secretory antibody is usually a polymeric antibody, preferably a polymeric IgA, comprising a complex of J-chain-containing polymer of Ig and secretory component (SC). The secretory component is a proteolytic cleavage product of the extracellular part of the polymeric immunoglobulin receptor (plgR) which binds to J-chain containing polymeric Ig. The secretory antibody is preferably a secretory IgA monoclonal antibody derived from a monoclonal antibody as defined above.

In some preferred embodiment, the neutralizing antibody is a recombinant human monoclonal antibody, preferably of IgG1 or IgA isotype. The IgA may be monomeric, polymeric or secretory IgA; it is preferably a polymeric or secretory IgA. In some more preferred embodiments, the recombinant antibody is a silent antibody that may further comprise mutations and/or or modifications to enhance antibody half-life, as described above.

The present invention also relates to an antigen binding fragment of an antibody that contain the variable domains comprising the CDRs domains as described above such as Fv, dsFv, scFv, Fab, Fab', F(ab')2. In particular, said antigen binding fragment is a F(ab')2 fragment. The F(ab')2 fragment can be produced by pepsin digestion of an antibody below the hinge disulfide; it comprises two Fab' fragments, and additionally a portion of the hinge region of the immunoglobulin molecule. Fab fragments are monomeric fragments obtainable by papain digestion of an antibody; they comprise the entire L chain, and a VH-CH1 fragment of the H chain, bound together through a disulfide bond. The Fab' fragments are obtainable from F(ab')2 fragments by cutting a disulfide bond in the hinge region. F(ab')2 fragments are divalent, i.e. they comprise two antigen binding sites, like the native immunoglobulin molecule; on the other hand, Fv (a VHVL dimer constituting the variable part of Fab), dsFv, scFv, Fab, and Fab' fragments are monovalent, i.e. they comprise a single antigen-binding site. These basic antigen-binding fragments of the disclosure can be combined together to obtain multivalent antigen-binding fragments, such as diabodies, tribodies or tetrabodies. These multivalent antigen-binding fragments are also part of the present invention. Fv fragments consist of the VL and VH domains of an antibody associated together by hydrophobic interactions; in dsFv fragments, the VH:VL heterodimer is stabilized by a disulphide bond; in scFv fragments, the VL and VH domains are connected to one another via a flexible peptide linker thus forming a single-chain protein.

In some particular embodiments, the antibody is a whole antibody or an antigen-binding fragment.

### Nucleic acid, host cell, antibody production

Also disclosed herein are nucleic acid molecule(s) that encode(s) the anti-sarbecovirus antibodies, in particular anti-SARS-CoV-2 antibodies, of the present disclosure.

Typically, said nucleic acid is recombinant, synthetic or semi-synthetic nucleic acid which is expressible in a host cell suitable for antibody expression or production, in particular human antibody production. The host cell may a cell for recombinant antibody production or a patient cell for antibody production *in vivo.* Typically, the nucleic acid may be DNA, RNA or mixed molecule, which may further be modified and/or included in any suitable expression vector. As used herein, the terms "vector" and "expression vector" mean the vehicle by which a DNA or RNA sequence (e.g. a foreign gene) can be introduced into a host cell, so as to transform the host and promote expression (e.g. transcription and translation) of the introduced sequence. The recombinant vector can be a vector for eukaryotic or prokaryotic expression, such as a plasmid, a phage for bacterium introduction, a YAC able to transform yeast, a viral vector and especially a retroviral vector, or any expression vector. An expression vector as defined herein is chosen to enable the production of an antibody, either *in vitro* or *in vivo.*

So, a further object of the disclosure relates to a vector comprising a nucleic acid as described herein.

Examples of nucleic acid molecules are those encoding the variable light and heavy chain amino acid sequences of the anti-SARS-CoV-2 antibody as disclosed in the previous section, and using the genetic code and, optionally taking into account the codon bias depending on the host cell species.

The nucleic acid molecule or construct sequence is advantageously codon-optimized for expression in a host cell suitable for antibody production in host cell, in particular mammalian cells. Codon optimization is used to improve protein expression level in living organism by increasing translational efficiency of target gene. Appropriate methods and softwares for codon optimization in the desired host are well-known in the art and publically available (see for example the GeneOptimizer software suite in Raab et al., Systems and Synthetic Biology, 2010, 4, (3), 215-225).

The host cell for antibody production may be eukaryote or prokaryote cell. Prokaryote cell is in particular bacteria. Eukaryote cell includes yeast, insect cell and mammalian cell.

Nucleic acids encoding anti-Sarbecovirus antibodies, in particular anti-SARS-CoV-2 antibodies of the disclosure with nucleotide sequences having at least 80%, for example, at least 85%, 90%, 95%, 96%, 97%, 98%, or 99% identity to any one of the above defined nucleotides sequences are also part of the present disclosure.

According to preferred embodiments, nucleic acids encoding anti-HR2 antibodies, or antigen-binding fragments thereof, of the present disclosure, comprise a nucleic acid sequence having at least 90% identity to at least one of **SEQ ID NO: 19** or **20.**

The present disclosure also pertains to nucleic acid molecules that derive from the latter sequences having been optimized for protein expression in host cells, in particular eukaryotic cells, preferably mammalian cells, for example, CHO or HEK cell lines or human cells.

In some embodiments, said nucleic acid molecule is a eukaryotic, preferably mammalian, expression cassette, wherein the antibody coding sequence(s) is operably linked to appropriate regulatory sequence(s) for their expression in an antibody producing cell or a patient cell. Such sequences which are well-known in the art include in particular a promoter, and further regulatory sequences capable of further controlling the expression of a transgene, such as without limitation, enhancer, terminator and intron. The promoter may be a tissue-specific, ubiquitous, constitutive or inducible promoter that is functional in the antibody producing cell. Such promoters are well-known in the art and their sequences are available in public sequence data bases.

In some particular embodiments, the nucleic acid is RNA, preferably mRNA, wherein the coding sequence of the antibody light and/or heavy chain is operably linked to appropriate regulatory sequence(s) for their expression in an individual's target cells or tissue(s). mRNA therapy is well-known in the art. mRNA is delivered into the host cell cytoplasm where expression generates the therapeutic protein of interest. mRNA construct comprises a cap structure, 5' and 3'untranslated regions (UTRs), and open reading frame (ORF), and a 3'poly(A) tail. mRNA construct may be non-replicating mRNA (MRM) or self-amplifying mRNA (SAM). SAM comprises the inclusion of genetic replication machinery derived from positive-strand mRNA viruses, most commonly alphaviruses such as Sindbis and Semliki-Forest viruses. In SAM constructs, the ORF encoding viral structural protein is replaced by the transcript encoding the therapeutic protein of interest, and the viral RNA-dependent RNA polymerase is retained to direct cytoplasmic amplification of the replicon construct. Trans-replicating RNA are disclosed for example in WO 2017/162461. RNA replicon from alphavirus suitable for gene expression are disclosed in WO 2017/162460. mRNA manufacturing process uses plasmid DNA (pDNA) containing a DNA-dependent RNA polymerase promoter, such as T7, and the corresponding sequence for the mRNA construct. The pDNA is linearized to serve as a template for the DNA-dependent RNA polymerase to transcribe the mRNA, and subsequently degraded by a DNase process step. The addition of the 5'cap and the 3'poly(A) tail can be achieved during the *in vitro* transcription step or enzymatically after transcription. Enzymatic addition of the cap can be accomplished by using guanylyl transferase and 2'-O-methyltransferase to yield a Cap0(^{N7Me}GpppN) or Cap1 (^{N7Me}GpppN^{2'-oMe}) structure, respectively, while the poly-A tail can be achieved through enzymatic addition via poly-A polymerase. mRNA is then purified using standard methods suitable for mRNA purification such as high-pressure liquid chromatography (HPLC) and others. Methods for producing mRNA are disclosed for example in WO 2017/182524.

To improve translation efficiency in treated subject cells, the mRNA according to the invention comprises a sequence which is codon-optimized for expression in human. Further improvements of the mRNA construct according to the invention to improve its stability and translation efficiency *in vivo* include optimization the length and regulatory element sequences of 5'-UTR and 3'UTR; base and/or sugar modifications in the cap structure to increase ribosomal interaction and/or mRNA stability; and modified nucleosides. Modified nucleosides may be in the 5'-UTR, 3'-UTR or ORF. Examples of modified nucleosides include pseudouridine and N-1-methylpseudouridine that remove intracellular signalling triggers for protein kinase R activation. Examples of modified nucleosides that reduce RNA degradation into cells are disclosed in WO 2013/039857. Modified cap structures are disclosed in WO 2011/015347 and WO 2019/175356. Optimized 3'-UTR sequences are disclosed in WO 2017/059902. Modified polyA sequences which improve RNA stability and translation efficiency are disclosed in US 2020/0392518. Modified mRNA with improved stability and translation efficiency are also disclosed in WO 2007/036366.

The invention may use any vector suitable for the delivery and expression of nucleic acid into individual's cells, in particular suitable for nucleic acid therapy. Such vectors that are well-known in the art include viral and non-viral vectors. Non-viral vector includes the various (non-viral) agents which are commonly used to either introduce or maintain nucleic acid into individual's cells. Agents which are used to introduce nucleic acid into individual's cells by various means include in particular polymer-based, particle-based, lipid-based, peptide-based delivery vehicles or combinations thereof, such as with no limitations cationic polymer, dendrimer, micelle, liposome, lipopolyplex, exosome, microparticle and nanoparticle including lipid nanoparticle (LNP) and viral-like particles; and cell penetrating peptides (CPP). Agents which are used to maintain nucleic acid into individual's cells include in particular naked nucleic acid vectors such as plasmids, transposons and mini-circles. Viral vectors are by nature capable of penetrating into cells and delivering nucleic acid(s) of interest into cells, according to a process named as viral transduction. As used herein, the term "viral vector" refers to a non-replicating, non-pathogenic virus engineered for the delivery of genetic material into cells. In viral vectors, viral genes essential for replication and virulence are replaced with an expression cassette for the transgene of interest. Thus, the viral vector genome comprises the transgene expression cassette flanked by the viral sequences required for viral vector production. As used herein, the term "recombinant virus" refers to a virus, in particular a viral vector, produced by standard recombinant DNA technology techniques that are known in the art. As used herein, the term "virus particle" or "viral particle" is intended to mean the extracellular form of a non-pathogenic virus, in particular a viral vector, composed of genetic material made from either DNA or RNA surrounded by a protein coat, called the capsid, and in some cases an envelope derived from portions of host cell membranes and including viral glycoproteins. As used herein, a viral vector refers to a viral vector particle. These vectors have minimal eukaryotic sequences to minimize the possibility of chromosomal integration. In addition, these approaches can advantageously be combined to introduce and maintain the nucleic acid of the invention into individual's cells.

In some embodiments, a mRNA according to the present invention as disclosed above is combined with a nucleic-acid delivery agent suitable for delivery of mRNA into mammalian host cells that are well-known in the art. The mRNA delivery agent may be a polymeric carrier, polycationic protein or peptide, lipid nanoparticle or other. For example, the mRNA (non-replicating or self-amplifying) may be delivered into cells using polymers, in particular cationic polymers, such as polyethylenimine (PEI), poly-L-Lysin (PEL), polyvinylamine (PVA) or polyallylamine (PAA), wherein the mRNA is preferentially present in the form of monomers, dimers, trimers or oligomers as disclosed in WO 2021/001417. Alternatively, the mRNA may be combined with polyalkyleneimine in the form of polyplex particles, suitable for intramuscular administration as disclosed in WO 2019/137999 or WO 2018/011406. The mRNA may also be combined with a polycation, in particular protamine, as disclosed in WO 2016/000792. One or more mRNA molecules may be formulated within a cationic lipid nanoparticle (LNP); for example the formulation may comprise 20-60% cationic lipid; 5-25% non-cationic lipid, 25-55% sterol and 0.5-15% PEG-modified lipid as disclosed WO 2015/164674. The mRNA may also be formulated in RNA decorated particles such as RNA decorated lipid particles, preferably RNA decorated liposomes as disclosed in WO 2015/043613.

In particular embodiments, the vector is a particle or vesicle, in particular lipid-based micro- or nano-vesicle or particle such as liposome or lipid nanoparticle (LNP). In more particular embodiments, the nucleic acid is RNA, in particular mRNA and the vector is a particle or vesicle, in particular LNP as described above. The LNP: mRNA mass ratio can be around 10:1 to 30:1.

In other embodiments, the nucleic acid is DNA, preferably included in an expression vector such as plasmid or viral vector. The invention also relates to a vector comprising the nucleic acid according to the present disclosure. Preferably, the vector is a recombinant integrating or non-integrating viral vector. Examples of recombinant viral vectors include, but not limited to, vectors derived from retrovirus, adenovirus, adeno-associated virus (AAV), herpes virus, poxvirus, and other virus. Retrovirus includes in particular lentivirus vector such as human immunodeficiency virus, including HIV type 1 (HIV-1) and HIV type 2 (HIV-2) vectors.

The polynucleotide according to the disclosure is prepared by the conventional methods known in the art. For example, it is produced by amplification of a nucleic sequence by PCR or RT-PCR, by screening genomic DNA libraries by hybridization with a homologous probe, or else by total or partial chemical synthesis. The recombinant vectors are constructed and introduced into host cells by the conventional recombinant DNA and genetic engineering techniques, which are known in the art.

A further object of the present disclosure relates to a host cell which has been transfected, infected or transformed by a nucleic acid and/or a vector according to the invention. As used herein, the term "transformation" means the introduction of a "foreign" (i.e. extrinsic or extracellular) gene, DNA or RNA sequence to a host cell, so that the host cell will express the introduced gene or sequence to produce a desired substance, typically a protein or enzyme coded by the introduced gene or sequence. The transformation may be transient or stable over time. Stable transformation may be by integration of the nucleic acid into the host cell genome. A host cell that receives and expresses introduced DNA or RNA bas been "transformed".

Said host cells may be prokaryotic cells such as bacteria or eukaryotic cells such as yeasts, insect cells or mammalian cells. Mammalian cells may be simian, human, dog and rodent cells. Mammalian host cells for expressing the antibodies of the disclosure include in particular Chinese Hamster Ovary (CHO cells) including dhfr- CHO cells (described in Urlaub and Chasin, 1980) used with a DHFR selectable marker (as described in Kaufman and Sharp, 1982), CHOK1 dhfr+ cell lines, NSO myeloma cells, COS cells and SP2 cells, for example GS CHO cell lines together with GS Xceed^{™} gene expression system (Lonza), HEK-293 cells (ATCC CRL-1573). In a preferred embodiment, said host cells are CHO cells, or HEK-293.

The polynucleotide, vector or cell of the disclosure are useful for the production of the protein of the invention using well-known recombinant DNA techniques. The polynucleotide or vector are also useful for nucleic acid therapy as disclosed below.

Antibodies of the present disclosure can be produced in a host cell transfectoma using, for example, a combination of recombinant DNA techniques and gene transfection methods as is well known in the art (Morrison, 1985). For expression of the light and heavy chains, the expression vector(s) encoding the heavy and light chains is transfected into a host cell by standard techniques. The various forms of the term "transfection" are intended to encompass a wide variety of techniques commonly used for the introduction of exogenous DNA into a prokaryotic or eukaryotic host cell, e.g., electroporation, calcium-phosphate precipitation, DEAE-dextran transfection and the like. When recombinant expression vectors encoding antibody genes are introduced into host cells, in particular eukaryotic cells such as mammalian cells, the antibodies are produced by culturing the host cells for a period of time sufficient for expression of the antibody in the host cells and, optionally, secretion of the antibody into the culture medium in which the host cells are grown. Antibodies can be recovered and purified for example from the culture medium after their secretion using standard protein purification methods (Shukla et al., 2007).

### Pharmaceutical composition and therapeutic use

In some embodiments, the nucleic acid is mRNA, preferably modified mRNA as disclosed herein; the mRNA including modified mRNA is advantageously formulated in a particle or vesicle, in particular LNP, as disclosed herein.

As used herein, the term "pharmaceutically acceptable" means approved by a regulatory agency or recognized pharmacopeia such as European Pharmacopeia, for use in animals and/or humans. The term "excipient" refers to a diluent, adjuvant, carrier, or vehicle with which the therapeutic agent is administered.

Any suitable pharmaceutically acceptable carrier, diluent or excipient can be used in the preparation of a pharmaceutical composition (See e.g., Remington: The Science and Practice of Pharmacy, Alfonso R. Gennaro (Editor) Mack Publishing Company, April 1997). Pharmaceutical compositions are typically sterile and stable under the conditions of manufacture and storage. Pharmaceutical compositions may be formulated as solutions (e.g. saline, dextrose solution, or buffered solution, or other pharmaceutically acceptable sterile fluids), microemulsions, liposomes, or other ordered structure suitable to accommodate a high product concentration (e.g. microparticles or nanoparticles). The carrier may be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as mannitol, sorbitol, or sodium chloride in the composition.

In some embodiments, the pharmaceutical composition is for systemic, local or systemic combined with local administration. Parenteral pharmaceutical composition includes a composition suitable for intravenous, intraarterial, subcutaneous, intraperitoneal or intramuscular administration. Local administration is preferably respiratory such as by nasal administration, inhalation, insufflation. or broncho-alveolar lavage. The administration may be parenteral injection or infusion, local delivery, or inhalation or sustained delivery. Preferably, the administration is by injection, inhalation, or injection combined with inhalation. Preferably the injection is intravenous, subcutaneous or intramuscular. The inhalation is advantageously done by nebulisation.

In some embodiments the pharmaceutical composition comprises a preservative. Non-limiting examples of preservatives are anti-oxidation agents and anti-bacterial agents. In some embodiments the preservative is present at a known concentration. The presence of a preservative in the composition distinguishes the composition from any composition that occurs in nature. It also imbues the composition with unique functions that are not present in any naturally occurring composition, such as in certain embodiments the ability to be used therapeutically under certain conditions in which the preservative is useful.

In some embodiments the pharmaceutical composition comprises a defined concentration of a recombinant human antibody of this invention. Such compositions do not occur naturally and have a different structure than any naturally occurring composition. The known concentration of the recombinant antibody imbues the compositions with unique functions that are not present in any naturally occurring composition, such as in certain embodiments the ability to be used therapeutically under certain conditions in which the defined concentration of the recombinant antibody is useful.

In some embodiments, a pharmaceutical composition comprising IgA, in particular polymeric or secretory IgA as disclosed herein is used for mucosal application, in particular to the respiratory tract, preferably by nebulisation or inhalation. Pharmaceutical composition comprising IgA, in particular polymeric (e.g., J-chain dimerization of IgA) or secretory IgA are preferred as prophylactic treatment, to prevent a Sarbecovirus infection, in particular a SARS-CoV-2 infection.

In some embodiments, a pharmaceutical composition comprising IgG, preferably IgG1, is used for injection, in particular intravenous, subcutaneous or intramuscular.

These pharmaceutical compositions are exemplary only and do not limit the pharmaceutical compositions suitable for other parenteral and non-parenteral administration routes. The pharmaceutical compositions described herein can be packaged in single unit dosage or in multidosage forms.

Preferably, the pharmaceutical compositions contain vehicles, which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. Sustained release formulations, such as PLA or PLGA or other polymers, for inhalation or injection can be used.

In certain aspects, the disclosure relates to an antibody, antigen-binding fragment thereof, or pharmaceutical composition according to any one of the preceding embodiments, for use as a medicament.

In certain aspects, the disclosure provides the therapeutic use of an antibody, antigen-binding fragment thereof or a composition according to any one of the preceding embodiments, preferably for treating, preventing or alleviating the symptoms of a Sarbecovirus-related disease, in particular a SARS-CoV-2-associated or -mediated disorder in a subject in need thereof.

The term "subject" or "patient" as used herein, refers to mammals. Mammalian species that can benefit from the disclosed methods of treatment include, but are not limited to, humans, non-human primates such as apes, chimpanzees, monkeys, and orangutans, domesticated animals, including dogs and cats, as well as livestock such as horses, cattle, pigs, sheep, and goats, or other mammalian species including, without limitation, mice, rats, guinea pigs, rabbits, hamsters, and the like.

As used herein, the term "treatment", "treat" or "treating" refers to any act intended to ameliorate the health status of patients such as therapy, prevention, prophylaxis and retardation of the disease. In certain embodiments, such term refers to the amelioration or eradication of a disease or symptoms associated with a disease, such as according to the present disclosure the reduction of the viral burden and/or levels of inflammation in the lungs. In other embodiments, this term refers to minimizing the spread or worsening of the disease resulting from the administration of one or more therapeutic agents to a subject with such a disease.

In a further aspect, the disclosure relates to a method of treating and/or reducing the risk of developing Sarbecovirus-associated disease, in particular a SARS-CoV-2-associated disorder, in a subject in need thereof that comprises administering to the subject a therapeutically effective amount of an antibody, an antigen-binding fragment thereof, or combination thereof, a nucleic acid or vector, or a pharmaceutical composition as described above.

In some embodiments, the subject is not infected with SARS-CoV-2 and the treatment is a prophylactic treatment.

In some particular embodiments, the method is a method of reducing the risk of developing a Sarbecovirus-associated disease, in particular a SARS-CoV-2-associated COVID-19 disease, wherein the risk of hospitalization or the risk of death is reduced by the treatment. In some particular embodiments, the reduction of the risk of developing a Sarbecovirus-associated disease, in particular a SARS-CoV-2 associated COVID-19 disease lasts at least 3 or 4 months, preferably 5 or 6 months, more preferably 7 to 9 months, after administration to the subject of a therapeutically effective amount of an antibody, an antigen-binding fragment thereof, or combination thereof, a nucleic acid or vector, or a pharmaceutical composition as described above.

In some embodiments, the subject is a COVID-19 patient and the treatment is a curative treatment.

In some particular embodiments, the method is a method of treating a Sarbecovirus-associated disease, in particular a SARS-CoV-2-associated COVID-19 disease, wherein the likelihood of developing severe disease is reduced by the treatment; wherein the likelihood of hospitalization is reduced by the treatment; wherein the subject is hospitalized.

In some particular embodiments, the method is a method of treating Sarbecovirus-associated disease, in particular a SARS-CoV-2-associated COVID-19 disease, wherein the subject is at risk of developing a SARS, more particularly a subject with concurrent underlying conditions such as obesity, diabetes, cancer, under immunosuppressive therapy, primary immune deficiency or unresponsive to vaccines. Non-limiting examples of subjects with concurrent underlying conditions include a subject receiving anti-CD20 antibody therapy, a subject having a lymphoid hemopathy, a solid organ transplant recipient or an allogeneic hematopoietic stem cell transplant recipient.

In the context of the invention, an "effective amount" means a therapeutically effective amount. As used herein a "therapeutically effective amount" refers to an amount effective, at dosages and for periods of time necessary to achieve the desired therapeutic result, such as prophylaxis, or treatment of Sarbecovirus-infection SARS-CoV-2-infection and in particular the reduction of the viral burden and/or levels of inflammation in the lungs. The therapeutically effective amount of the product of the invention, or pharmaceutical composition that comprises it may vary according to factors such as the disease state, age, sex, and weight of the individual, and the ability of the product or pharmaceutical composition to elicit a desired response in the individual. Dosage regimens may be adjusted to provide the optimum therapeutic response. A therapeutically effective amount is also typically one in which any toxic or detrimental effect of the product or pharmaceutical composition is outweighed by the therapeutically beneficial effects.

The product of the disclosure will be typically included in a pharmaceutical composition or medicament, optionally in combination with a pharmaceutical carrier, diluent and/or adjuvant. Such composition or medicinal product comprises the product of the disclosure in an effective amount, sufficient to provide a desired therapeutic effect, and a pharmaceutically acceptable carrier or excipient.

In one embodiment the antibody or antigen-binding fragment or the pharmaceutical composition for its therapeutic use is administered to the subject or patient by a parenteral route, in particularly by intravenous, intraarterial, subcutaneous, intraperitoneal, or intramuscular route. In another particular embodiment, the antibody or antigen-binding fragment or the pharmaceutical composition for its therapeutic use is administered to the subject or patient by inhalation.

The amount of product of the invention that is administered to the subject or patient may vary depending on the particular circumstances of the individual subject or patient including, age, sex, and weight of the individual; the nature and stage of the disease, the aggressiveness of the disease; the route of administration; and/or concomitant medication that has been prescribed to the subject or patient. Dosage regimens may be adjusted to provide the optimum therapeutic response.

For any particular subject, specific dosage regimens may be adjusted over time according to the individual needs and the professional judgment of the person administering or supervising the administration of the compositions. Dosage ranges set forth herein are exemplary only and do not limit the dosage ranges that may be selected by medical practitioners.

In one embodiment, the antibody or antigen-binding fragment according to the disclosure can be administered to the subject or patient for the treatment of SARS-CoV-2 associated disease in an amount or dose comprised within a range of 2.5 mg/kg to 40 mg/kg (kg: subject's or patient's body weight). In a more particular embodiment, the antibody or antigen-binding fragment is administered in an amount comprised within a range of 5 to 30 mg/kg. In a more particular embodiment, the antibody or antigen-binding fragment is administered in an amount comprised within a range of 8.5 to 28.5 mg/kg for a person weighing 70 kg. In a more particular embodiment, the antibody or antigen-binding fragment is administered at a dosage of at least 5 mg/kg, preferably 10 mg/kg, more preferably 15 mg/kg, and more preferably 30 mg/kg.

In some embodiments, the pharmaceutical composition is included in a kit that may further comprise instructions or packaging materials that describe how to administer the product contained within the kit to a patient. Containers of the kit can be of any suitable material, e.g., glass, plastic, metal, etc., and of any suitable size, shape, or configuration. In certain embodiments, the kits may include one or more ampoules or syringes that contain the products of the invention in a suitable liquid or solution form.

Another aspect of the invention relates to a medical device, comprising the pharmaceutical composition according to the present disclosure. The medical device is in a form suitable for the administration of the composition. In some embodiments, the medical device is suitable for the injection of the composition; said medical device is advantageously chosen from a syringe, infusion bag, injection port, and others that are well-known in the art. In other embodiments, the medical device is suitable for the respiratory tract administration of the composition; said medical device is advantageously chosen from an inhaler, a nebulizer, such as small-volume nebulizer, and others that are well-known in the art.

Another aspect of the invention relates to the use of a pharmaceutical composition according to the present disclosure for the manufacture of a medicament for the prevention or treatment of a Sarbecovirus infection or related disease, and in particular of a SARS-CoV-2 infection and associated COVID-19 disease.

### Detection, diagnostic reagents, kits and methods

The combinations of anti-RBD and anti-HR2 antibodies or fragment thereof comprising the antigen-binding site according to the present disclosure are useful as reagent for the detection or diagnosis of a Sarbecovirus infection, in particular a SARS-CoV-2 infection, or contamination in various samples, including in particular biological or environmental samples.

The sample is any sample suspected of containing a Sarbecovirus, in particular a SARS-CoV-2, such as in particular biological or environmental samples. A biological sample may be any tissue, body fluid or stool. Non-limiting examples of body fluids include whole-blood, serum, plasma, urine, cerebral spinal fluid (CSF), and mucosal secretions, such as with no limitations oral and respiratory tract secretions (sputa, saliva and the like). Samples include swabs such as oral or nasopharyngeal (NP) swabs, aspirate, wash or lavage. Samples for detection and diagnostic tests for Sarbecoviruses, in particular SARS-CoV-2, can be taken from the upper (nasopharyngeal/oropharyngeal swabs, nasal aspirate, nasal wash or saliva) or lower respiratory tract (sputum or tracheal aspirate or bronchoalveolar lavage (BAL).

Preferred biological samples include nasopharyngeal swab and saliva sample. Samples also include environmental samples that may contain SARS-CoV-2 such as air, water, soil, food, beverages, feed, water (e.g., fresh water, salt water, waste water, and drinking water), sewage, sludge, environmental surfaces and others. The environmental surface sample is for example a surface swab or swipe.

The detection or diagnosis is performed by immunoassay technique which is well-known in the art and rely on the detection of antigen-antibody complexes using an appropriate label. The method of the invention may use any immunoassay such as with no limitations, immunoblotting, immunoprecipitation, ELISA, immunocytochemistry or immunohistochemistry, and immunofluorescence like flow cytometry assay, and FACS. Flow cytometry, also known as flow virometry, nanoscale flow cytometry or simply small-particle flow cytometry is a rapid, high-throughput, and effective method to quantify intact viral particles released by an infected cell.

The invention encompasses a method for the detection of a Sarbecovirus, in particular of a SARS-CoV-2, in a sample comprising: contacting said sample with an anti-HR2 antibody, or combination of an anti-HR2 and of an anti-RBD antibody, or antigen-binding fragment thereof, according to the present disclosure and detecting the antigen-antibody complexes formed.

According to preferred embodiments, the method for detecting a Sarbecovirus includes at least one step of detecting:
- the formation of a complex between the anti-HR2 antibody of the disclosure and the HR2 epitope in the sample; and/or
- the formation of a complex between the anti-RBD antibody of the disclosure and the RBD epitope in the sample.

According to some embodiments, the method for detecting a Sarbecovirus includes a step of detecting the formation of a ternary complex between the anti-HR2 antibody of the disclosure, the anti-RBD antibody of the disclosure and a polypeptide comprising the HR2 epitope and the RBD epitope.

The method of the invention may use any appropriate label used in immunoassays such as enzymes, chemiluminescent, fluorescent dyes/proteins or radioactive agents, or others. The label may be on the antibody or fragment thereof which binds to the antigen or on a binding-partner such as secondary antibody or avidin/streptavidin conjugated to a label.

The antibody is preferably labelled, in the form of a conjugate or fusion protein, and the antigen-antibody complexes are detected by measuring the signal from the label by any appropriate means available for that purpose as disclosed above.

In some embodiments, antigen detection is performed by ELISA, lateral flow immunoassay, or bead-based immunoassay.

The detection step may be qualitative or semi-quantitative, and may comprise detecting the presence or level of viral antigen in the sample. In some embodiments, the detecting step comprises the determination of the amount of bound antigen in the mixture, and optionally, comparing the amount of bound antigen in the mixture with at least one predetermined value.

The detection of the presence or level of viral antigen in a biological sample from an individual using the methods of the invention is indicative of whether the individual is suffering from a Sarbecovirus infection or related disease; in particular of a SARS-CoV-2 infection or associated COVID-19 disease.

Therefore, the above method of the invention is useful for the diagnosis of a Sarbecovirus infection, or related disease, in particular a SARS-CoV-2 infection or associated COVID-19 disease in an individual, as well as monitoring of treatment in a patient, such as a COVID-19 patient.

The treatment may be an antiviral treatment or immunotherapeutic treatment using neutralizing antibodies, in particular SARS-CoV-2 neutralizing antibodies.

In some embodiments, the above method is a method of diagnosis comprising the step of deducing therefrom whether the individual is suffering from a Sarbecovirus infection or related disease, in particular a SARS-CoV-2 infection or associated disease.

The detection of an antigen-antibody complex as defined above, and its comparison with a reference value, may thus be indicative of the occurrence of a Sarbecovirus in the individual and/or the occurrence of a Sarbecovirus-related disease in the individual.

In some embodiments, the above method is a method of monitoring of treatment in a patient, for example a COVID-19 patient, comprising the step of deducing therefrom whether the treatment is efficient is not. Treatment efficacy is determined by a decrease of viral antigen level compared to previous viral antigen level determined in the patient, before treatment or during the treatment course.

In some embodiments in connection with this aspect of the invention, the above method of diagnosis comprises a further step of administering an appropriate treatment to the individual depending on whether or not the individual is diagnosed with Sarbecovirus infection or related disease, in particular a SARS-CoV-2 virus infection, and in particular COVID-19 associated disease.

In some embodiments in connection with this aspect of the invention, the above method of monitoring of treatment in a patient, for example a COVID-19 patient, comprises a further step of modifying the COVID-19 treatment when said treatment is determined as not being efficient in the patient.

In another aspect, the disclosure further relates to a kit for the detection or diagnosis of a Sarbecovirus infection, in particular a SARS-CoV-2 infection or contamination, comprising at least an anti-HR2 antibody or antigen-binding fragment thereof, or combination thereof comprising an anti-HR2 and an anti-RBD according to the disclosure, preferably further including a detectable label.

The kit optionally comprises reagents for the detection of the antigen/antibody complex. Reagents available for this purpose are well-known in the art and include with no limitation buffers, secondary antibody conjugated to a label, avidin/streptavidin conjugated to a label. In some preferred embodiments of the kit of the invention, the antibody, and optional reagents are in lyophilised form to allow ambient storage. The components of the kits are packaged together into any of the various containers suitable for antigen/antibody complex detection such as plates, slides, wells, dishes, beads, particles, cups, strands, chips, strips and others. The kit optionally includes instructions for performing at least one specific embodiment of the method of the invention. In some advantageous embodiments, the kit comprises micro-well plates or microtubes, preferably in a dried format, i.e., wherein the wells of the plates or microtubes comprise a dried composition containing at least the antibody, and preferably further comprising all the reagents for the detection of antigen/antibody complex. In some other advantageous embodiments, the antibody and optional reagents are included into any of the devices available for immunoassay.

The practice of the present invention will employ, unless otherwise indicated, conventional techniques, which are within the skill of the art. Such techniques are explained fully in the literature.

The invention will now be exemplified with the following examples, which are not limitative, with reference to the attached drawings.

### FIGURE LEGENDS

**Figure 1****. Binding characteristics and SARS-CoV-2 neutralizing capacities of anti-HR2 antibody Cv2.3132.**
   **(A)** Graph comparing the ELISA binding of Cv2.3132 IgG antibody to SARS-CoV-2 tri-S, tri-S2 and S1 proteins (left), and spike trimers from other coronaviruses (a-coronaviruses: SARS-CoV-1, MERS-CoV, HKU1, and β-coronaviruses: OC43, 229E) (right). SARS-CoV-2 BA.1 tri-S was also included. Means ± SD of duplicate values are shown.
   **(B)** Diagram presenting the sequences of the 20-mer overlapping 5-amino acid peptides covering the entire SARS-CoV-2 HR2 region (n=8). The most immunoreactive peptide bound by Cv2.3132 is highlighted In blue.
   **(C)** Graph comparing the ELISA binding of Cv2.3132 IgG to FP (K22K) and HR2 peptides. Means ± SD of duplicate values are shown.
   **(D)** Graphs showing the ELISA reactivity of Cv2.3132 IgG against 20-mer HR2 overlapping 5-amino acid peptides (n=8). Means ± SD of triplicate values are shown.
   **(E)** Diagram presenting the sequences of the 20-mer overlapping 5-amino acid peptides covering the entire SARS-CoV-2 HR2 region (n=8). The most immunoreactive peptide bound by Cv2.3132 is highlighted.
   **(F)** Heatmap showing the flow cytometric binding of Cv2.3132 IgG to spike-expressing 293-F cells for SARS-CoV-2 variants (SARS-CoV-2, D614G, [VOCs]: α, β, γ, δ, BA.1, BA.2, BA.4/5, [VOIs]: ε, ι, k, λ, µ). Geometric means of duplicate log₁₀ΔMFI values are shown in each cell.
   **(G)** Graphs comparing the *in vitro* neutralizing activity of Cv2.3194, Cv2.3132 and combined antibodies (Cv2.3194 + Cv2.3132) against selected SARS-CoV-2 variants as determined with the S-Fuse neutralization assay. Error bars indicate the SD of values from 2 independent experiments.
   **(H)** Heatmap comparing the IC₅₀ values for the SARS-CoV-2 neutralization calculated from (G) for Cv2.3194, Cv2.3132 and combined antibodies (Combo).
   **(I)** diagram showing the experimental design of Cv2.3132 antibody treatment in K18-hACE2 mice infected intranasally (i.n.) with 10⁴ PFU of the SARS-CoV-2 variant β (B.1.351) (left). Animals were either pre-treated 6h before infection with ~ 10 mg/kg (1 mg) of Cv2.3132 IgG or treated 6h post-infection with ~ 10 mg/kg (1 mg) of Cv2.3132 IgG or mGO53 isotype control (ctr) by intraperitoneal (i.p.) injection. Graphs showing the evolution of initial body weight (% Δ weight, middle) and survival rate (right) in animal groups. Groups of mice were compared in the Kaplan-Meier analysis using Log-rank Mantel-Cox test.

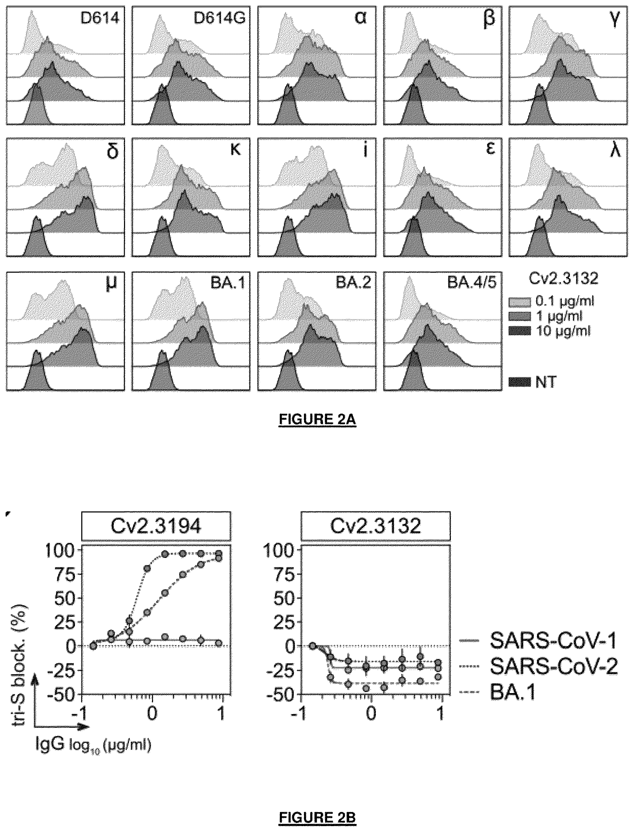

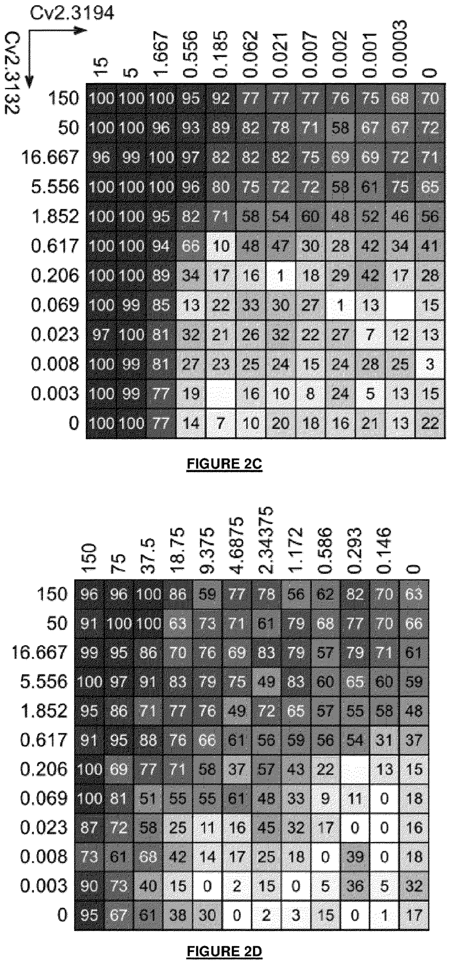

**Figure 2****. Cv2.3132 binding and neutralization properties.**
   **(A)** Representative flow cytometric histograms comparing the binding of Cv2.3132 IgG (tested at a concentration 0.1, 1 and 10 µg/ml) to spike-expressing 293-F cells for SARS-CoV-2 variants (SARS-CoV-2, D614G, [VOCs]: α, β, γ, δ, BA.1, BA.2, BA.4/5, [VOIs]: ε, ι, κ, λ, µ). NT, non-transfected cell control.
   **(B)** Graphs comparing the tri-S-ACE2 blocking capacity of Cv2.3132 IgG with class I anti-RBD antibody Cv2.3194 against SARS-CoV-2, BA.1 and SARS-CoV-1 as determined by ELISA. Means ± SD of duplicate values are shown.
   **(C-D)** Dose-response neutralization matrices showing the neutralizing activities of Cv2.3194 and Cv2.3132 alone and combined against BA.4.6 and XBB.1.5. IC₅₀ values for each combination are indicated in the cells.

### EXAMPLES

### Material & Methods

### Viruses and recombinant viral proteins

The SARS-CoV-2 viral stocks for the following strains were prepared and titrated in the Viral and Immunity Unit (Institut Pasteur) as previously described in Planas et al. ("Resistance of Omicron subvariants BA.2.75.2, BA.4.6, and BQ.1.1 to neutralizing antibodies"; Nat. Commun. 2023; 14(1):824): reference D614G strain (hCoV-19/France/GE1973/2020, GISAID ID: EPI_ISL_414631; National Reference Center for Respiratory Viruses (Institut Pasteur)), BA.2.75.2 (GISAID ID ID: EPI_ISL_15731524) as previously described in **Planas et al.,** BQ.1.1 (GISAID ID: EPI_ISL_15731523), BA.4.6 (GISAID ID: EPI_ISL_15729633), XBB.1 (hCoV-19/France/PAC-HCL022171892001/2022)) and XBB.1.5 (GISAID EPI_ISL_16353849)). The Beta strain (β, B.1.351; hcoV-19/France/IDF-IPP00078/2021) used for mouse experiments was supplied by the National Reference Centre for Respiratory Viruses (Institut Pasteur, France). All work with infectious virus was performed in biosafety level 3 containment laboratories at Institut Pasteur.

Trimeric SARS-CoV-2, SARS-CoV-1, MERS-CoV, OC43-CoV, HKU1-CoV, 229E-CoV, NL63-CoV, BA.1 ectodomains (tri-S); SARS-CoV-2 tri-S2 protein, S1 subunit, and human angiotensin-converting enzyme 2 (ACE2) ectodomain cloned into pcDNA3.1/Zeo(+) vector were produced by transient transfection of Freestyle 293-F cells (Thermo Fisher Scientific), and purified by high-performance chromatography using the Ni Sepharose^{®} Excel Resin (GE Healthcare) as previously described in Planchais et al. ("Potent human broadly SARS-CoV-2-neutralizing IgA and IgG antibodies effective against Omicron BA.1 and BA.2"; J. Exp. Med. 2022; 219(7):e20220638).

### ELISAs

ELISAs were performed as previously described in **Planchais et al.** Briefly, high-binding 96-well ELISA plates (Costar, Corning) were coated overnight with 250 ng/well of purified recombinant Coronavirus proteins. After washings with 0.05% Tween 20-PBS (washing buffer), plates were blocked 2 h with 2% BSA, 1 mM EDTA, 0.05% Tween 20-PBS (Blocking buffer), washed, and incubated with serially diluted purified IgG mAbs in PBS. Recombinant IgG1 antibodies were tested at 10 µg/ml, and 7 consecutive 1:4 dilutions in PBS. After washings, the plates were revealed by incubation for 1 h with goat HRP-conjugated anti-human IgG (Jackson ImmunoReseach, 0.8 µg/ml final) and by adding 100 µl of HRP chromogenic substrate (ABTS solution, Euromedex) after washing steps. Optical densities were measured at 405nm (OD₄₀₅ₙₘ), and background values given by incubation of PBS alone in coated wells were subtracted. Experiments were performed using HydroSpeed^{™} microplate washer and Sunrise^{™} microplate absorbance reader (Tecan Männedorf, Switzerland). For peptide-ELISA, binding of SARS-CoV-2 and control IgG antibodies to the fusion peptide FP (KRSFIEDLLFNKVTLADAGFIK), HR2 peptide (DVDLGDISGINASVVNIQKEIDRLNEVAKNLNESLIDLQELGKYEQYIKWP), and 20-mer HR2 overlapping 5-amino acid peptides (n=8) (GenScript Biotech, 500 ng/well) was tested using the same procedure as previously described in Planchais et al. ("Potent human broadly SARS-CoV-2-neutralizing IgA and IgG antibodies effective against Omicron BA.1 and BA.2"; J. Exp. Med. 2022; 219(7):e20220638).

For the competition experiments of tri-S-binding to ACE2, ELISA plates (Costar, Corning) were coated overnight with 250 ng/well of purified ACE2 ectodomain. After washings, plates were blocked 2 h with Blocking buffer, PBST-washed, and incubated with recombinant IgG1 mAbs at 10 µg/ml and 7 consecutive 1:2 dilutions in presence of biotinylated tri-S protein at 1 µg/ml in PBS. After washings, the plates were revealed by incubation for 30 min with streptavidin HRP-conjugated (BD Biosciences) as described above.

### Flow cytometry binding assay

To evaluate spike cross-reactivity, Freestyle^{™} 293-F were transfected with pUNO1-Spike-dfur expression vectors (Spike and SpikeV1 to V12 plasmids, Invivogen) (1.2 µg plasmid DNA per 106 cells) using PEI-precipitation method. Forty-eight hours post-transfection, 0.5x106 transfected and non-transfected control cells were incubated with IgG antibodies for 30 min at 4°C (0.1, 1 and 10 µg/ml). After washings, cells were incubated 20 min at 4°C with AF647-conjugated goat anti-human IgG antibodies (1:1000 dilution; Thermo Fisher Scientific) and LIVE/DEAD Fixable Viability dye Aqua (1:1000 dilution; Thermo Fisher Scientific), washed and resuspended in PBS-Paraformaldehyde 1% (Electron Microscopy Sciences). Data were acquired using a CytoFLEX flow cytometer (Beckman Coulter), and analyzed using FlowJo software (v10.7.1; FlowJo LLC). Antibodies were tested in duplicate.

### Infrared immunoblotting

Recombinant tri-S proteins (Wuhan tri-S, BA.1 tri-S and tri-S2) were heat-denatured at 100°C for 3 min in loading buffer (Invitrogen) containing 1X sample reducing agent (Invitrogen), and 0.5% SDS. Nitrocellulose membranes were hydrated with PBS and inserted into a Miniblot apparatus (Immunetics). Native and denatured tri-S proteins (10 µg total) were incubated on membranes for 2 h in PBS, and saturated in 5% dry milk-PBS-0.05% Tween 20 (PBST) overnight at 4°C. Membranes were rotated 90° clockwise, inserted into a Miniblot apparatus and then incubated for 2 h with human IgG antibodies (at a concentration ranging from 0.08 to 10 µg/ml) and a mouse anti-Hisx6 antibody (1 µg/ml, BD Biosciences) in 5% dry milk-PBST in each channel. After PBST washings, membranes were incubated for 1 h with 1:25,000-diluted Alexa Fluor 680-conjugated donkey anti-human IgG (Jackson ImmunoResearch) and 1:25,000-diluted IR Dye^{®} 800CW-conjugated goat anti-mouse IgG (LI-COR Biosciences) in 5% dry milk-PBST. Finally, membranes were washed and scanned using the iBright^{™} FL1500 Imaging System (Invitrogen, Thermo Fisher Scientific).

### SARS-CoV-2 S-Fuse neutralization assay

S-Fuse cells (1:1 mix of U2OS-ACE2-GFP1-10 and U2OS-ACE2-GFP11) were prepared and plated at a density of 2 × 10⁴ per well in a µClear 96-well plate (Greiner Bio-One) as previously described in Planas et al. ("Resistance of Omicron subvariants BA.2.75.2, BA.4.6, and BQ.1.1 to neutralizing antibodies"; Nat. Commun. 2023; 14(1):824).

SARS-CoV-2 VOC viruses (MOI 0.1) were incubated with recombinant IgG1 mAbs at 100 µg/ml, and 17 consecutive 1:2 dilutions in culture medium for 30 min at room temperature and added to S-Fuse cells. The cells were fixed, 18 h later, in 4% paraformaldehyde, washed and stained with Hoechst stain (dilution 1 :10,000; Invitrogen). Images were acquired with an Opera Phenix high-content confocal microscope (Perkin Elmer). The area displaying GFP expression and the number of nuclei were quantified with Harmony software 4.8 (Perkin Elmer). The percentage neutralization was calculated from the number of syncytia as follows: 100 × (1 - (value with IgG - value in "non-infected") / (value in "no IgG"-value in "non-infected")). IC₅₀ values were calculated using Prism software (v.9.3.1, GraphPad Prism Inc.) by fitting replicate values using the four-parameters dose-response model (variable slope). For evaluating the synergistic potential of combined antibodies (Cv2.3194+Cv2.3132), S-Fuse neutralization experiments were performed using BA.4.6 and XBB.1.5 strains as described above but using mixed antibodies at different concentration ratio in comparison to the single antibodies alone (0). For BA.4.6, Cv2.3132 at 150 µg/ml and 10 three-fold dilutions was mixed with Cv2.3134 at 15 µg/ml and 10 three-fold dilutions. For XBB.1.5, Cv2.3132 at 150 µg/ml and 10 three-fold dilutions was mixed with Cv2.3134 at 150 µg/ml and 10 two-fold dilutions. The % neutralization was calculated for each combination. Two independent experiments were performed, and mean values were calculated.

The quantification of the synergistic effect of the two antibodies, i.e. an anti-RBD and an anti-HR2 of the present disclosure, in the cocktail was calculated using the SynergyFinder web application (v3.0) (https://synerqyfinder.fimm.fi) as described in lanevski et al. ("SynergyFinder 3.0: an interactive analysis and consensus interpretation of multi-drug synergies across multiple samples"; Nucleic Acids Res. 2022; 50(W1):W739-W743), with the corrected Zero interaction potency (ZIP) model. A global synergy score < -10 indicates antagonism, ranging from -10 to 10 indicates an additive effect, and < 10 indicates synergy.

### SARS-CoV-2 infection and treatment in K18-hACE2 mice

B6.Cg-Tg(K18-ACE2)2Prlmn/J mice (stock #034860) were imported from The Jackson Laboratory (Bar Harbor, ME, USA), and bred at the Institut Pasteur under strict SPF conditions. Infection studies were performed on 6 to 16 wk-old male and female mice, in animal biosafety level 3 (BSL-3) facilities at the Institut Pasteur, in Paris. All animals were handled in strict accordance with good animal practice. Animal work was approved by the Animal Experimentation Ethics Committee (CETEA 89) of the Institut Pasteur (project dap 200008 and 200023) and authorized by the French legislation (under project 24613) in compliance with the European Communities Council Directives (2010/63/UE, French Law 2013-118, February 6, 2013) and according to the regulations of Pasteur Institute Animal Care Committees before experiments were initiated. Anesthetized (ketamine/xylazine) mice were inoculated intranasally (i.n.) with 1 ×10⁴ PFU of SARS-CoV-2 Virus Beta 3.1.351 (20 µl/nostril). Six hours pre- or post-inoculation, mice received an intraperitoneal (i.p.) injection of 10 mg/kg of Cv2.3132 or mGO53 control IgG antibody. Clinical signs of disease (ruffled fur, hunched posture, reduced mobility and breathing difficulties) and weight loss were monitored during 16 days. Mice were euthanized when they reached pre-defined end-point criteria. Sera were extracted from blood collected by puncture of the retromandibular vein. Mouse survival were compared across groups using a Kaplan-Meier analysis and Log-rank Mantel-Cox test (GraphPad Prism, v8.2, GraphPad Prism Inc.).

### Example 1. Identification of a monoclonal antibody targeting the HR2 region in a convalescent donor.

From a convalescent donor, we identified an IgG antibody targeting the S2 spike region **(****Fig.1A****),** Cv2.3132, which displayed SARS-CoV-2 neutralization potential. Cv2.3132 cross-reacted with the recombinant spike trimers of SARS-CoV-1, but not of other α-and β-coronaviruses (**Fig.1A**). Cv2.3132 bound a non-conformational dependent S2 epitope, which we identified here as the heptad repeat 2 (HR2) region (**Fig.1C**). Mapping analysis with HR2-overlapping peptides shows that Cv2.3132 recognized mainly, but weakly compared to the entire HR2 peptide (D1163-P1213), the peptide E1188-E1207 in the C-terminal portion of the SARS-CoV-2 HR2 (**Fig.1D** and **Fig. 1E**), a highly conserved region across Sarbecoviruses including SARS-CoV-2 variants.

In agreement, Cv2.3132 had comparable binding to spike-expressing cells from VOCs α, β, γ, δ, BA.1, BA.2, BA.4/5, and VOIs ε, ι, κ, λ, µ (**Fig.1F**)**.** Cv2.3132 neutralized *in vitro* SARS-CoV-2 D614G (IC₅₀ = 35.4 µg/ml), and the more recent variants BA.4.6, BQ.1.1, XBB.1 and XBB.1.5 SARS-CoV-2 variants with IC₅₀ values ranging from 4.8 to 8.3 µg/ml in the S-Fuse neutralization assay, but reaching a neutralization plateau at ~ 50% (**Fig.1G** and **1H**)**.** In contrast, BA.2.75.2 virus was resistant to Cv2.3132 antibody neutralization (**Fig.1G**), most likely due to the D1199N substitution present in the HR2 C-terminal region of this variant (**Fig.1E**). Considering the location of Cv2.3132 epitope on the S2 subunit, and the inability of Cv2.3132 in blocking the spike-ACE-2 interactions by ELISA (**Fig.1C**), we suspect that the antibody may interfere with membrane fusion mechanisms.

### Example 2. Cooperative and/or synergistic effects of an anti-HR2 and of an anti-RBD

Cooperative and/or synergistic effects of antibody cocktails made of anti-RBD and anti-S2 (FP or stem helix) neutralizers have been suggested.

Therefore, we next evaluated whether combining Cv2.3132 with Cv2.3194 would lead to additive or synergistic effects. The antibody Cv2.3194 is as described in WO 2022228827**.**

First, we compared the *in vitro* neutralizing activity of an equimolar Cv2.3132-Cv2.3194 IgG mixture with single antibodies. We found that the Cv2.3132-Cv2.3194 antibody combination exhibited superior neutralizing capacities than single antibodies against BA.4.6, BQ.1.1, XBB.1 and XBB.1.5 (IC₅₀ range: 0.05-2.1 µg/ml) (**Fig.1G** and **1H**). Combined antibodies did not neutralize BA.2.75.2 as efficiently as Cv2.3194 alone due to the lack of neutralizing activity of Cv2.3132 against this variant (**Fig.1G** and **1H**). Synergy-scoring model analysis of *in vitro* neutralization experiments made with BA.4.6 and XBB.1.5 viruses showed that high local synergistic effects were observed at certain antibody concentrations. Thus, for instance, highest δ-scores for XBB.1.5 neutralization were obtained in the range of 0.6-2.3 µg/ml and 0.21-1.85 µg/ml for Cv2.3194 and Cv2.3132, respectively.

To determine whether Cv2.3132 is active *in vivo* against SARS-CoV-2 infection, we tested its prophylactic and therapeutic activity against SARS-CoV-2 VOC Beta in K18-hACE2 transgenic mice as previously described. A single administration of Cv2.3132 IgG antibodies at ~10 mg/kg (0.25 mg *i.p*.) 6h prior to (pre-exposure) or after (therapy) infection with 10⁴ PFU of SARS-CoV-2 Beta (β) protected 37.5% of the animals from death (*vs* 0% in the control group; p=0.003 and p=0.0007, respectively (**Fig.1I**).

Together, these data indicate that anti-HR2 antibody Cv2.3132 is a broad Sarbecovirus neutralizing antibody, active *in vivo,* and efficiently blocking SARS-CoV-2 infection with the most recent VOCs when combined with the resilient class I anti-RBD antibody Cv2.3194 isolated from the same donor.

**SEQUENCE LISTING**

| **SEQ ID** | **Description** | **Sequence** |
|---|---|---|
| **1** | Cv2.3132 - CDR-H1 | DYYMS |
| **2** | Cv2.3132 - CDR-H2 | YISSSGSVQYYADSVKG |
| **3** | Cv2.3132 - CDR-H3 | YYYDSSGYWKRLDY |
| **4** | Cv2.3132 - CDR-L1 | RASQSISRYLN |
| **5** | Cv2.3132 - CDR-L2 | AASSLQS |
| **6** | Cv2.3132 - CDR-L3 | QQSYSTPLT |
| **7** | Cv2.3132 - H-FW1 | EVQLVESGGGLVKPGGSLRLSCAASGFTFS |
| **8** | Cv2.3132 - H-FW2 | WIRQAPGKGLEWVS |
| **9** | Cv2.3132 - H-FW3 | RFTISRDNAKNSLYLQMNSLRAEDTAVYYCAR |
| **10** | Cv2.3132 - H-FW4 | WGQGTLVTVSS |
| **11** | Cv2.3132 - L-FW1 | DIQMTQSPSSLSASVGDRVTITC |
| **12** | Cv2.3132 - L-FW2 | WYQQKPGKAPKLLIY |
| **13** | Cv2.3132 - L-FW3 | GVPSRFSGSGSGTDFTLTISSLQPEDFATYHC |
| **14** | Cv2.3132 - L-FW4 | FGGGTKVEIK |
| **15** | Cv2.3132 - VH | EVQLVESGGGLVKPGGSLRLSCAASGFTFSDYYMSWIRQAP GKGLEWVSYISSSGSVQYYADSVKGRFTISRDNAKNSLYLQ MNSLRAEDTAVYYCARYYYDSSGYWKRLDYWGQGTLVTVS S |
| **16** | Cv2.3132 - VL | DIQMTQSPSSLSASVGDRVTITCRASQSISRYLNWYQQKPG KAPKLLIYAASSLQSGVPSRFSGSGSGTDFTLTISSLQPEDFA TYHCQQSYSTPLTFGGGTKVEIK |
| **17** | Cv2.3132 - full length IgG light chain | DIQMTQSPSSLSASVGDRVTITCRASQSISRYLNWYQQKPG KAPKLLIYAASSLQSGVPSRFSGSGSGTDFTLTISSLQPEDFA TYHCQQSYSTPLTFGGGTKVEIKRTVAAPSVFIFPPSDEQLK SGTASVVCLLNNFYPREAKVQWKVDNALQSGNSQESVTEQ DSKDSTYSLSSTLTLSKADYEKHKVYACEVTHQGLSSPVTKS FNRGEC |
| **18** | Cv2.3132 - full length IgG heavy chain | EVQLVESGGGLVKPGGSLRLSCAASGFTFSDYYMSWIRQAP GKGLEWVSYISSSGSVQYYADSVKGRFTISRDNAKNSLYLQ MNSLRAEDTAVYYCARYYYDSSGYWKRLDYWGQGTLVTVS SASTKGPSVFPLAPSSKSTSGGTAALGCLVKDYFPEPVTVS WNSGALTSGVHTFPAVLQSSGLYSLSSVVTVPSSSLGTQTYI CNVNHKPSNTKVDKRVEPKSCDKTHTCPPCPAPELLGGPSV FLFPPKPKDTLMISRTPEVTCVVVDVSHEDPEVKFNWYVDG VEVHNAKTKPREEQYNSTYRVVSVLTVLHQDWLNGKEYKC KVSNKALPAPIEKTISKAKGQPREPQVYTLPPSREEMTKNQV SLTCLVKGFYPSDIAVEWESNGQPENNYKTTPPVLDSDGSF FLYSKLTVDKSRWQQGNVFSCSVMHEALHNHYTQKSLSLSP GK |
| **19** | Cv2.3132 - VL_nucleotide | GACATCCAGATGACCCAGTCTCCATCCTCCCTGTCTGCAT CTGTAGGAGACAGAGTCACCATCACTTGCCGGGCAAGTC AGAGCATTAGCAGGTATTTAAATTGGTATCAGCAGAAACC AGGGAAAGCCCCTAAGCTCCTGATCTATGCTGCATCCAGT TTGCAAAGTGGGGTCCCATCAAGGTTCAGTGGCAGTGGA TCTGGGACAGATTTCACGCTCACCATCAGCAGTCTGCAAC CTGAAGATTTTGCAACTTACCACTGTCAACAGAGTTACAG TACCCCGCTCACTTTCGGCGGAGGGACCAAGGTGGAGAT CAAA |
| **20** | Cv2.3132 - VH_nucleotide | GAGGTGCAGCTGGTGGAGTCTGGGGGAGGCTTGGTCAA GCCTGGAGGGTCCCTGAGACTCTCCTGTGCAGCCTCTGG ATTCACCTTCAGTGACTACTACATGAGCTGGATCCGCCAG GCTCCAGGGAAGGGGCTGGAGTGGGTTTCATACATTAGT AGTAGTGGTAGTGTCCAATACTACGCAGACTCTGTGAAGG GCCGATTCACCATCTCCAGGGACAACGCCAAGAACTCTCT GTATCTGCAAATGAACAGCCTGAGAGCCGAGGACACGGC CGTGTATTACTGTGCGAGGTATTACTATGATAGTAGTGGT TATTGGAAAAGGTTGGACTACTGGGGCCAGGGAACCCTG GTCACCGTCTCCTCA |
| **21** | HR2 Peptide 1 | DVDLGDISGINASVVNIQKE |
| **22** | HR2 Peptide 2 | DISGINASVVNIQKEIDRLN |
| **23** | HR2 Peptide 3 | NASVVNIQKEIDRLNEVAKN |
| **24** | HR2 Peptide 4 | NIQKEIDRLNEVAKNLNESL |
| **25** | HR2 Peptide 5 | IDRLNEVAKNLNESLIDLQE |
| **26** | HR2 Peptide 6 | EVAKNLNESLIDLQELGKYE |
| **27** | HR2 Peptide 7 | LNESLIDLQELGKYEQYIKW |
| **28** | HR2 Peptide 8 | IDLQELGKYEQYIKWP |
| **29** | Cv2.1169 - HCDR1 | TSAVQ |
| **30** | Cv2.1169 - HCDR2 | WIVVGSGNTNYAQKFQE |
| **31** | Cv2.1169 - HCDR3 | PYCSGGTCLDGFDI |
| **32** | Cv2.1169 - LCDR1 | RASQSVSRSYLA |
| **33** | Cv2.1169 - LCDR2 | SASSRAT |
| **34** | Cv2.1169 - LCDR3 | QQYGSSPWT |
| **35** | Cv2.1353 - HCDR1 | SNYMN |
| **36** | Cv2.1353 - HCDR2 | VIYSGGSTFYADSVKG |
| **37** | Cv2.1353 - HCDR3 | DLGPMGFDI |
| **38** | Cv2.1353 - LCDR1 | RASQGISSYLA |
| **39** | Cv2.1353 - LCDR2 | AASTLQS |
| **40** | Cv2.1353 - LCDR3 | QQLNSDPPVT |
| **41** | Cv2.3194 - HCDR1 | SNYMS |
| **42** | Cv2.3194 - HCDR2 | VIYPGGSTFYADSVKG |
| **43** | Cv2.3194 - HCDR3 | DLVVYGMDV |
| **44** | Cv2.3194 - LCDR1 | RASQSVSSSYLA |
| **45** | Cv2.3194 - LCDR2 | GASSRAT |
| **46** | Cv2.3194 - LCDR3 | QQGVT |
| **47** | Cv2.3235 - HCDR1 | RNYMS |
| **48** | Cv2.3235 - HCDR2 | VIYSGGSTFYADSVKG |
| **49** | Cv2.3235 - HCDR3 | DGDYYGMDV |
| **50** | Cv2.3235 - LCDR1 | RASQGISSFLA |
| **51** | Cv2.3235 - LCDR2 | GASTLQS |
| **52** | Cv2.3235 - LCDR3 | QRLDSYPPIT |
| **53** | Cv2.5213 - HCDR1 | SNYMS |
| **54** | Cv2.5213 - HCDR2 | LIYSGGSTYYADSVKG |
| **55** | Cv2.5213 - HCDR3 | DLNYYGMDV |
| **56** | Cv2.5213 - LCDR1 | RASQGISSYLA |
| **57** | Cv2.5213 - LCDR2 | AASTLQS |
| **58** | Cv2.5213 - LCDR3 | QQLDSYSPFT |
| **59** | Cv2.5179 - HCDR1 | SSAVQ |
| **60** | Cv2.5179 - HCDR2 | WIVVGSGNTNYAQKFQE |
| **61** | Cv2.5179 - HCDR3 | PHCSSTSCYDAFDI |
| **62** | Cv2.5179 - LCDR1 | RASQSVSSSYLA |
| **63** | Cv2.5179 - LCDR2 | GASSRAT |
| **64** | Cv2.5179 - LCDR3 | QQYGRSPWT |
| **65** | Heptad-Repeat 2 (HR2) region 1163-1213 | DVDLGDISGINASVVNIQKEIDRLNEVAKNLNESLIDLQELGKY EQYIKWP |
| **66** | Fusion peptide | KRSFIEDLLFNKVTLADAGFIK |

## Claims

1. A combination of:
(i) An isolated antibody, or antigen-binding fragment thereof, directed against the viral Spike protein heptad repeat 2 (HR2) of a Sarbecovirus, in particular of Severe Acute Respiratory Syndrome-related Coronavirus 2 (SARS-CoV-2); and
(ii) An isolated antibody, or antigen-binding fragment thereof, directed against the viral Spike protein receptor binding-domain (RBD) of a Sarbecovirus, in particular of Severe Acute Respiratory Syndrome-related Coronavirus 2 (SARS-CoV-2);
**characterized in that** the isolated antibody or antigen-binding fragment thereof directed against the viral Spike protein heptad repeat 2 (HR2) of a Sarbecovirus, binds specifically to a polypeptide comprising **SEQ ID NO: 26** within the polypeptide **SEQ ID NO: 65.**

2. The combination according to claim 1, wherein the (i) isolated antibody, or antigen-binding fragment thereof, directed against the viral Spike protein heptad repeat 2 (HR2) of a Sarbecovirus, in particular of Severe Acute Respiratory Syndrome-related Coronavirus 2 (SARS-CoV-2) is **characterized in that** it comprises:
- a heavy chain variable domain comprising all three of: a heavy chain CDR1 of SEQ ID NO: 1, a heavy chain CDR2 of SEQ ID NO: 2 and a heavy chain CDR3 of SEQ ID NO: 3; and a light chain variable domain comprising all three of: a light chain CDR1 of SEQ ID NO: 4, a light chain CDR2 of SEQ ID NO: 5 and a light chain CDR3 of SEQ ID NO: 6.

3. The combination according to claim 1 or 2, wherein the (i) isolated antibody, or antigen-binding fragment thereof, directed against the viral Spike protein heptad repeat 2 (HR2) of a Sarbecovirus, in particular of Severe Acute Respiratory Syndrome-related Coronavirus 2 (SARS-CoV-2) is **characterized in that** it comprises a heavy chain comprising the amino acid sequence of **SEQ ID NO: 15** and (ii) a light chain comprising the amino acid sequence of **SEQ ID NO: 16.**

4. The combination according to claim 1 or 2 or 3; **characterized in that** the antibody, or antigen-binding fragment thereof, directed against the viral Spike protein heptad repeat 2 (HR2) binds specifically to polypeptide consisting of **SEQ ID NO: 26.**

5. The combination according to any one of the preceding claims, wherein the (ii) isolated antibody, or antigen-binding fragment thereof, directed against the viral Spike protein receptor binding-domain (RBD) of a Sarbecovirus, in particular of Severe Acute Respiratory Syndrome-related Coronavirus 2 (SARS-CoV-2), is **characterized in that** it comprises:
- a heavy chain variable domain comprising all three of: a heavy chain CDR1 of SEQ ID NO: 29, a heavy chain CDR2 of SEQ ID NO:30 and a heavy chain CDR3 of SEQ ID NO: 31; and a light chain variable domain comprising all three of: a light chain CDR1 of SEQ ID NO: 32, a light chain CDR2 of SEQ ID NO: 33 and a light chain CDR3 of SEQ ID NO: 34; or
- a heavy chain variable domain comprising all three of: a heavy chain CDR1 of SEQ ID NO: 35, a heavy chain CDR2 of SEQ ID NO:36 and a heavy chain CDR3 of SEQ ID NO: 37; and a light chain variable domain comprising all three of: a light chain CDR1 of SEQ ID NO: 38, a light chain CDR2 of SEQ ID NO: 39 and a light chain CDR3 of SEQ ID NO: 40; or
- a heavy chain variable domain comprising all three of: a heavy chain CDR1 of SEQ ID NO: 41, a heavy chain CDR2 of SEQ ID NO: 42 and a heavy chain CDR3 of SEQ ID NO: 43; and a light chain variable domain comprising all three of: a light chain CDR1 of SEQ ID NO: 44, a light chain CDR2 of SEQ ID NO: 45 and a light chain CDR3 of SEQ ID NO: 46; or
- a heavy chain variable domain comprising all three of: a heavy chain CDR1 of SEQ ID NO: 47, a heavy chain CDR2 of SEQ ID NO: 48 and a heavy chain CDR3 of SEQ ID NO: 49; and a light chain variable domain comprising all three of: a light chain CDR1 of SEQ ID NO: 50, a light chain CDR2 of SEQ ID NO: 51 and a light chain CDR3 of SEQ ID NO: 52; or
- a heavy chain variable domain comprising all three of: a heavy chain CDR1 of SEQ ID NO: 53, a heavy chain CDR2 of SEQ ID NO: 54 and a heavy chain CDR3 of SEQ ID NO: 55; and a light chain variable domain comprising all three of: a light chain CDR1 of SEQ ID NO: 56, a light chain CDR2 of SEQ ID NO: 57 and a light chain CDR3 of SEQ ID NO: 58; or
- a heavy chain variable domain comprising all three of: a heavy chain CDR1 of SEQ ID NO: 59, a heavy chain CDR2 of SEQ ID NO:60 and a heavy chain CDR3 of SEQ ID NO: 61; and a light chain variable domain comprising all three of: a light chain CDR1 of SEQ ID NO: 62, a light chain CDR2 of SEQ ID NO: 63 and a light chain CDR3 of SEQ ID NO: 64.

6. The combination according to any one of the preceding claims, wherein:
(i) the isolated antibody, or antigen-binding fragment thereof, directed against the viral Spike protein heptad repeat 2 (HR2) of a Sarbecovirus, in particular of Severe Acute Respiratory Syndrome-related Coronavirus 2 (SARS-CoV-2) is as defined in claim 2; and
(ii) the isolated antibody, or antigen-binding fragment thereof, directed against the viral Spike protein receptor binding-domain (RBD) of a Sarbecovirus, in particular of Severe Acute Respiratory Syndrome-related Coronavirus 2 (SARS-CoV-2) is as defined in claim 5.

7. The combination according to any one of the preceding claims, wherein:
(i) the isolated antibody, or antigen-binding fragment thereof, directed against the viral Spike protein heptad repeat 2 (HR2) of a Sarbecovirus, in particular of Severe Acute Respiratory Syndrome-related Coronavirus 2 (SARS-CoV-2) is as defined in claim 2; and
(ii) the isolated antibody, or antigen-binding fragment thereof, directed against the viral Spike protein receptor binding-domain (RBD) of a Sarbecovirus, in particular of Severe Acute Respiratory Syndrome-related Coronavirus 2 (SARS-CoV-2) is **characterized in that** it comprises:
- a heavy chain variable domain comprising all three of: a heavy chain CDR1 of SEQ ID NO: 41, a heavy chain CDR2 of SEQ ID NO: 42, and a heavy chain CDR3 of SEQ ID NO: 43; and a light chain variable domain comprising all three of: a light chain CDR1 of SEQ ID NO: 44, a light chain CDR2 of SEQ ID NO: 45 and a light chain CDR3 of SEQ ID NO: 46

8. An isolated nucleic acid or expression vector or host cell coding for:
(i) an antibody, or antigen-binding fragment thereof, directed against the viral Spike protein heptad repeat 2 (HR2) of a Sarbecovirus, in particular of Severe Acute Respiratory Syndrome-related Coronavirus 2 (SARS-CoV-2); preferably comprising at least a nucleic acid sequence encoding the heavy and/or light chain of said antibody or antigen-binding fragment thereof; and
(ii) an antibody, or antigen-binding fragment thereof, directed against the viral Spike protein receptor binding-domain (RBD) of a Sarbecovirus, in particular of Severe Acute Respiratory Syndrome-related Coronavirus 2 (SARS-CoV-2) preferably comprising at least a nucleic acid sequence encoding the heavy and/or light chain of said antibody or antigen-binding fragment thereof;
**characterized in that** the antibody or antigen-binding fragment thereof directed against the viral Spike protein heptad repeat 2 (HR2) of a Sarbecovirus, binds specifically to a polypeptide comprising **SEQ ID NO: 26** within the polypeptide **SEQ ID NO: 65.**

9. A kit comprising
(i) an antibody or antigen-binding fragment thereof, directed against the viral Spike protein heptad repeat 2 (HR2) of a Sarbecovirus, in particular of Severe Acute Respiratory Syndrome-related Coronavirus 2 (SARS-CoV-2), or nucleic acid or expression vector or host cell coding for the said antibody or antigen-binding fragment thereof; and
(ii) an antibody or antigen-binding fragment thereof, directed against the viral Spike protein receptor binding-domain (RBD) of a Sarbecovirus, in particular of Severe Acute Respiratory Syndrome-related Coronavirus 2 (SARS-CoV-2), or nucleic acid or expression vector or host cell coding for the said antibody or antigen-binding fragment thereof;
**characterized in that** the isolated antibody or antigen-binding fragment thereof directed against the viral Spike protein heptad repeat 2 (HR2) of a Sarbecovirus, binds specifically to a polypeptide comprising **SEQ ID NO: 26** within the polypeptide **SEQ ID NO: 65.**

10. A pharmaceutical composition comprising the combination according to any one of claims 1 to 7, or nucleic acid or vector or host cell according to claim 8; and a pharmaceutically acceptable carrier.

11. The combination according to any one of claims 1 to 7, or the nucleic acid or vector or host cell according to claim 8, or the kit according to claim 9, or the pharmaceutical composition according to claim 10; for use as a medicament.

12. The combination according to any one of claims 1 to 7, or the nucleic acid or vector according to claim 8, or the kit according to claim 9, or the pharmaceutical composition according to claim 10; for use in a method for preventing and/or reducing the likelihood of occurrence and/or treating a Sarbecovirus infection and related disease, in particular a SARS-CoV-2 infection and associated COVID-19 disease.

13. An isolated antibody, or antigen-binding fragment thereof, directed against the viral Spike protein heptad repeat 2 (HR2) of a Sarbecovirus; for use in a method for preventing and/or reducing the likelihood of occurrence and/or treating a Sarbecovirus infection and related disease; **characterized in that** it comprises
- a heavy chain variable domain comprising all three of: a heavy chain CDR1 of SEQ ID NO: 1, a heavy chain CDR2 of SEQ ID NO: 2 and a heavy chain CDR3 of SEQ ID NO: 3; and a light chain variable domain comprising all three of: a light chain CDR1 of SEQ ID NO: 4, a light chain CDR2 of SEQ ID NO: 5 and a light chain CDR3 of SEQ ID NO: 6;
further **characterized in that** the isolated antibody or antigen-binding fragment thereof directed against the viral Spike protein heptad repeat 2 (HR2) of a Sarbecovirus, binds specifically to a polypeptide comprising **SEQ ID NO: 26** within the polypeptide **SEQ ID NO: 65.**

14. A nucleic acid or expression vector or host cell coding for an antibody, or antigen-binding fragment thereof, directed against the viral Spike protein heptad repeat 2 (HR2) of a Sarbecovirus according to the preceding claim; for use in a method for preventing and/or reducing the likelihood of occurrence and/or treating a Sarbecovirus infection and related disease.

15. The isolated antibody, or antigen-binding fragment thereof, according to claim 13, or the nucleic acid or vector or host cell according to claim 14; for use in a method for preventing and/or reducing the likelihood of occurrence and/or treating a SARS-CoV-2 infection and associated COVID-19 disease.

16. The isolated antibody, or antigen-binding fragment thereof for use according to claim 13, or the nucleic acid or vector or host cell for use according to claim 14, **characterized in that** the antibody is a human neutralizing antibody.

17. The isolated antibody, or antigen-binding fragment thereof for use according to claim 13, or the nucleic acid or vector or host cell for use according to claim 14, **characterized in that** the antibody is not a single-domain antibody.

18. An *in vitro* method for detecting a Sarbecovirus in a sample, comprising a step of contacting said sample with:
- at least one antibody, or antigen-binding fragment thereof, directed against the viral Spike protein heptad repeat 2 (HR2) of a Sarbecovirus, in particular of Severe Acute Respiratory Syndrome-related Coronavirus 2 (SARS-CoV-2); and
- at least of one antibody, or antigen-binding fragment thereof, directed against the viral Spike protein receptor binding-domain (RBD) of a Sarbecovirus, in particular of Severe Acute Respiratory Syndrome-related Coronavirus 2 (SARS-CoV-2);
wherein the isolated antibody or antigen-binding fragment thereof directed against the viral Spike protein heptad repeat 2 (HR2) of a Sarbecovirus, binds specifically to a polypeptide comprising **SEQ ID NO: 26** within the polypeptide **SEQ ID NO: 65.**
